(19)

Europäisches
Patentamt
European
Patent Office
Office européen
des brevets

(11) **EP 2 308 493 A1**

(12) **EUROPEAN PATENT APPLICATION**
published in accordance with Art. 153(4) EPC

(43) Date of publication:
**13.04.2011 Bulletin 2011/15**

(21) Application number: **09794426.8**

(22) Date of filing: **07.07.2009**

(51) Int Cl.:
*A61K 31/455* (2006.01)   *A61K 9/10* (2006.01)
*A61K 31/202* (2006.01)   *A61K 31/232* (2006.01)
*A61P 3/06* (2006.01)   *A61P 7/02* (2006.01)
*A61P 7/06* (2006.01)   *A61P 9/00* (2006.01)
*A61P 9/08* (2006.01)   *A61P 9/10* (2006.01)
*A61P 9/14* (2006.01)   *A61P 17/02* (2006.01)
*A61P 17/04* (2006.01)   *A61P 17/14* (2006.01)
*A61P 21/02* (2006.01)   *A61P 25/04* (2006.01)
*A61P 27/02* (2006.01)   *A61P 43/00* (2006.01)

(86) International application number:
**PCT/JP2009/062352**

(87) International publication number:
**WO 2010/004982 (14.01.2010 Gazette 2010/02)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **07.07.2008   JP 2008177315
27.03.2009   JP 2009079601**

(71) Applicant: **Mochida Pharmaceutical Co., Ltd.
Tokyo 160-8515 (JP)**

(72) Inventors:
• **ISHIKAWA Hiroshi
Tokyo 160-8515 (JP)**
• **KANEHIRO Hideo
Tokyo 160-8515 (JP)**
• **FUJII Hirosato
Tokyo 160-8515 (JP)**

(74) Representative: **Grünecker, Kinkeldey,
Stockmair & Schwanhäusser
Anwaltssozietät
Leopoldstrasse 4
80802 München (DE)**

(54) **AMELIORATING OR THERAPEUTIC AGENT FOR DYSLIPIDEMIA**

(57)   An ameliorating or therapeutic agent for dyslipidemia which is safe and has high effective and a method of using such an ameliorating or therapeutic agent are provided. The ameliorating or therapeutic agent for dyslipidemia includes at least one selected from the group consisting of EPA and pharmaceutically acceptable salts and esters thereof and nicotinic acid or a pharmaceutically acceptable derivative thereof in combination as active ingredients .

EP 2 308 493 A1

**Description**

TECHNICAL FIELD

The present invention provides an ameliorating or therapeutic agent for dyslipidemia and particularly hypercholesterolemia, hypertriglyceridemia, high low density lipoprotein cholesterolemia, high non-high density lipoprotein cholesterolemia, and low high density lipoprotein cholesterolemia, and a method of using the ameliorating or therapeutic agent.

BACKGROUND ART

Recently, with changes in Japanese lifestyle including changes in eating habits and lack of exercise, metabolic syndrome observed as obesity, dyslipidemia, diabetes and hypertension is increasing drastically. Metabolic syndrome may trigger vascular complications such as peripheral arterial disease, ischemic heart diseases and cerebral infarction and considerably reduce the quality of life all through life. Peripheral arterial disease is a disease caused by the lack of blood flow to a peripheral skin or muscle as a result of narrowing or obstruction of a blood vessel by arteriosclerosis or other inflammation due to any cause, for example, arteriosclerosis obliterans (hereinafter abbreviated as "ASO"), Buerger's disease, Raynaud's disease or Raynaud's syndrome, or strong constriction of a blood vessel. ASO is a disease in which an artery is narrowed or obstructed by arteriosclerosis to cause an ischemic condition, and accompanies intermittent claudication, pain during walking, joint pain, muscle fatigue, eye strain, numbness, coldness and cyanosis, which may develop into muscle atrophy, ischemic ulcer and necrosis. Mild cases are treated with vasodilators, anticoagulants or platelet aggregation inhibitors but severe cases require revascularization such as bypass grafting and in cases where a therapeutic effect is not obtained because of peripheral necrosis or pain at rest, the extremity of a patient is amputated (see Non-Patent Literature 1).

"Hyperlipidemia" which has conventionally been one disease of the metabolic syndrome was renamed "dyslipidemia" in 2007 in the Guidelines for Prevention of Atherosclerotic Cardiovascular Diseases (Non-Patent Literature 2). The total cholesterol (hereinafter abbreviated as "Cho") level was deleted from the diagnostic criteria and replaced by three types of diseases including high low density lipoprotein (hereinafter abbreviated as "LDL") cholesterolemia, low high density lipoprotein (hereinafter abbreviated as "HDL") cholesterolemia, and hypertriglyceridemia (triglyceride is hereinafter abbreviated as "TG"). This guideline reports drugs for treating dyslipidemia including an HMG-CoA reductase inhibitor (hereinafter referred to as "statin") for mainly reducing the Cho level, an anion exchange resin for adsorbing bile acid, probucol for hypercatabolism of LDL, a nicotinic acid derivative for suppressing the hormone-sensitive lipase activity, fibrate drugs and icosapentaenoic acid (hereinafter abbreviated as "EPA") for mainly reducing the TG level, and ezetimibe for inhibiting the absorption of Cho.

It is reported that the nicotinic acid derivative suppresses activation of hormone-sensitive lipase to control lipolysis in the peripheral adipose tissues and reduce the flow of free fatty acids into liver, as a result of which synthesis of lipoprotein in liver and catabolism of apoprotein AI are suppressed to show the effect of increasing the HDLCho level. On the other hand, it is known that the nicotinic acid derivative causes main side effects such as itching and hot flash due to peripheral vascular dilatation, may exacerbate the insulin resistance and is to be administered with care to patients with diabetes (see Non-Patent Literature 2).
Tocopherol nicotinate is a nicotinic acid derivative in which nicotinic acid is bound to tocopherol (vitamin E) and it is revealed that tocopherol nicotinate has more stable and longer-term pharmacological effects than the case where the two components are used in combination (for example improved lipid metabolism, activated microcirculating system, strengthened blood vessels, inhibited platelet aggregation and increased blood oxygen partial pressure) while showing the physiological effects of the two components (see Non-Patent Literature 3). Tocopherol nicotinate is commercially available in Japan as a drug for treating accompanying symptoms resulting from the following hypertension, hyperlipidemia and peripheral circulatory disturbance resulting from the following ASO (Juvela N (registered trademark) from Eisai Co., Ltd.).

Ethyl ester of EPA (hereinafter referred to as "EPA-E") is commercially available in Japan as a drug for improvement of the associatedulcer, pain and coldness in ASO and treating hyperlipidemia (Epadel from Mochida Pharmaceutical Co., Ltd.). EPA-E is reported to have the effect of reducing the TG level or increasing the HDLCho level and is effective in treating dyslipidemia. EPA-E is taken in the platelet membrane phospholipids to increase the content ratio of EPA to the membrane constituents to competitively inhibit the arachidonic acid metabolism of the platelet membrane, thus showing the effect of inhibiting the production of thromboxane A2 and platelet aggregation. In addition, this drug is also taken in the blood vessel walls to control the progression of arteriosclerosis by the action of retaining the arterial elasticity. It is revealed that this drug shows the effect of improving the blood flow by the action of inhibiting the platelet aggregation, retaining the arterial elasticity, and stabilizing the atherosclerotic plaque under the influence of the drug taken in the plaque on the number of macrophages and their function (see Non-Patent Literature 4).

In cases where the respective lipid levels do not reach the control target values by the use of a single drug for treating dyslipidemia, the dose of the single drug is increased or a combination therapy is considered. Particularly in the secondary prevention, there are cases in which an LDLCho level of less than 100 mg/dL cannot be reached with the single drug, and in such cases it is necessary to consider an effective combination therapy of various drugs. In the combination therapy, a method which can make full use of the characteristics of each drug and in which the safety is confirmed is selected.

Exemplary combination therapies published in Japan to be effective in controlling the serum lipids include (1) statin combined with resin, (2) statin combined with fibrate, (3) statin combined with probucol, (4) statin combined with a nicotinic acid derivative, (5) probucol combined with a nicotinic acid derivative, and (6) statin combined with probucol and resin. (4) Statin combined with a nicotinic acid derivative shows the effect of reducing the Cho level while increasing the HDLCho level. In the combination therapy, the most attention is to be paid to the occurrence of side effects such as rhabdomyolysis infrequently seen in the use of (2) statin combined with fibrate (see Non-Patent Literature 2).

With regard to the drug combination system, it is reported that various drugs including EPA-E and tocopherol nicotinate were simultaneously administered when sarpogrelate hydrochloride was administered to patients with ASO and hypertriglyceridemia but there is no mention on the action of EPA-E and tocopherol nicotinate (see Non-Patent Literature 5).

It is reported that, three months after the administration of simvastatin, EPA-E and tocopherol nicotinate to patients with percutaneous transluminal coronary angioplasty, there was no influence on the serum total Cho but the minimum blood vessel diameter was significantly large and restenosis rate was significantly low (see Non-Patent Literature 6), and that blood TG was reduced after the administration of bezafibrate, tocopherol nicotinate and EPA-E to patients hospitalized with acute pancreatitis due to hyperlipoproteinemia (see Non-Patent Literature 7).

CITATION LIST

NON-PATENT LITERATURE

Non-Patent Literature 1: Nanzando's Medical Dictionary, 19th edition, Nanzando Co., Ltd., March, 2006, 2265
Non-Patent Literature 2: Guideline for Prevention of Atherosclerotic Cardiovascular Diseases, 2007, edited by Japan Atherosclerosis Society, Kyowa Kikaku Ltd., April 25, 2007
Non-Patent Literature 3: Juvela N, Drug interview form, Eisai Co., Ltd., February, 2008
Non-Patent Literature 4: Epadel S, Drug interview form, Mochida Pharmaceutical Co., Ltd., March, 2007
Non-Patent Literature 5: Angiology Frontier, 2007, 6:171-175
Non-Patent Literature 6: Japanese Circulation Journal, 2000, 64, Suppl.: 309, speech subject: 0496
Non-Patent Literature 7: Abstract of the 40th Annual Congress of Japanese Society for Abdominal Emergency Medicine, 2004, 532, speech subject-233

SUMMARY OF INVENTION

TECHNICAL PROBLEMS

An object of the present invention is to provide an ameliorating or therapeutic agent for dyslipidemia which is of high safety, has high effective and is easy to use, the agent being used for ameliorating or treating dyslipidemia and particularly hypercholesterolemia, hypertriglyceridemia, high LDL cholesterolemia, high non-HDL cholesterolemia and low HDL-cholesterolemia, and for suppressing the progression of metabolic syndrome and cardio-cerebrovascular events as well as a method of using the ameliorating or therapeutic agent.

Another object of the present invention is to provide an ameliorating or therapeutic agent for ameliorating or treating peripheral arterial disease associated with dyslipidemia and particularly ASO, Buerger's disease, Raynaud's disease and Raynaud's disease syndrome, and an ameliorating or therapeutic agent for peripheral arterial disease which is used to control the development of peripheral ulcer of the extremities and gangrene, is of high safety, has high effective and is easy to use, as well as a method of using such an ameliorating or therapeutic agent.

SOLUTION TO PROBLEMS

The inventors of the present invention have made an intensive study to achieve the foregoing objects and as a result found that safety and other exceptional effects that have been unattainable by the single administration of the respective components are achieved by using at least one selected from the group consisting of EPA and pharmaceutically acceptable salts and esters thereof in combination with nicotinic acid or a pharmaceutically acceptable derivative thereof, and the present invention has been thus completed. In other words, the ameliorating or therapeutic agent for dyslipidemia or the ameliorating or therapeutic agent for peripheral arterial disease associated with dyslipidemia as provided by the present invention includes a combined drug in which at least one selected from the group consisting of EPA and pharmaceutically acceptable salts and esters thereof, and nicotinic acid or a pharmaceutically acceptable derivative thereof are used in combination as the active ingredients , and a combined composition. Exemplary embodiments of the present invention are described below.

(1) An ameliorating or therapeutic agent for dyslipidemia comprising, as active ingredients to be applied in combination, at least one compound selected from the group consisting of EPA and pharmaceutically acceptable salts and esters thereof, and nicotinic acid or a pharmaceutically acceptable derivative thereof.
(2) The ameliorating or therapeutic agent according to (1) wherein the dyslipidemia is at least one selected from the group consisting of hypercholesterolemia, hypertriglyceridemia, high LDL cholesterolemia, high very low density lipoprotein (hereinafter abbreviated as "VLDL") cholesterolemia, high non-HDL cholesterolemia, high intermediate density lipoprotein (hereinafter abbreviated as "LDL") cholesterolemia, hyper-free fatty acidemia, hyperphospholipidemia, hyperchylomicronemia, hyperapobetalipoproteinemia, hyperlipoproteinemia (a) (hereinafter abbreviated as "Lp(a)"), hyper-remnant-like particles (hereinafter abbreviated as "RLP") lipoproteinemia, high small dense (hereinafter abbreviated as "sd") LDL cholesterolemia, low HDL cholesterolemia, low very high density lipoprotein (hereinafter abbreviated as "VHDL") cholesterolemia, and peripheral arterial disease associated with dyslipidemia. (3) The ameliorating or therapeutic agent according to (1) or (2) wherein EPA-E is used for the EPA and the pharmaceutically acceptable salts and esters thereof.

(4) The ameliorating or therapeutic agent according to any one of (1) to (3) wherein the nicotinic acid or the pharmaceutically acceptable derivative thereof is at least one compound selected from the group consisting of nicotinic acid, nicotinic acid amide, tocopherol nicotinate, niceritrol, nicomol and inositol hexanicotinate.
(5) The ameliorating or therapeutic agent according to any one of (1) to (4) wherein the nicotinic acid or the pharmaceutically acceptable derivative thereof is nicotinic acid or tocopherol nicotinate.

(6) The ameliorating or therapeutic agent according to any one of (1) to (5) wherein EPA-E is used for the EPA and the pharmaceutically acceptable salts and esters thereof and nicotinic acid or tocopherol nicotinate is used for the nicotinic acid or the pharmaceutically acceptable derivative thereof.

(7) The ameliorating or therapeutic agent according to any one of (1) to (6) wherein the therapeutic effect obtained by using the at least one compound selected from the group consisting of the EPA and the pharmaceutically acceptable salts and esters thereof in combination with the nicotinic acid or the pharmaceutically acceptable derivative thereof is larger than the sum of the therapeutic effects obtained by singly using at least one compound selected from the group consisting of the EPA and the pharmaceutically acceptable salts and esters thereof, and the nicotinic acid or the pharmaceutically acceptable derivative thereof at the same doses as used in the case of the combined use.

(8) The ameliorating or therapeutic agent according to any one of (1) to (7) which is a composite formulation comprising the at least one compound selected from the group consisting of the EPA and the pharmaceutically acceptable salts and esters thereof, and the nicotinic acid or the pharmaceutically acceptable derivative thereof.

(9) The ameliorating or therapeutic agent according to any one of (1) to (7) which contains as its effective component at least one compound selected from the group consisting of the EPA and the pharmaceutically acceptable salts and esters thereof, the agent used to ameliorate or treat dyslipidemia of a patient to which the nicotinic acid or the pharmaceutically acceptable derivative thereof is administered.
(10) The ameliorating or therapeutic agent according to any one of (1) to (7) which contains as its effective component the nicotinic acid or the pharmaceutically acceptable derivative thereof, the agent used to ameliorate or treat dyslipidemia of a patient to which the at least one compound selected from the group consisting of the EPA and the pharmaceutically acceptable salts and esters thereof is administered.

(11) The ameliorating or therapeutic agent according to any one of (1) to (9) wherein the at least one compound selected from the group consisting of the EPA and the pharmaceutically acceptable salts and esters thereof is used in combination with the nicotinic acid or the pharmaceutically acceptable derivative thereof by administering the at least one compound selected from the group consisting of the EPA and the pharmaceutically acceptable salts and esters thereof to a patient to which the nicotinic acid or the pharmaceutically acceptable derivative thereof is administered.

(12) The ameliorating or therapeutic agent according to any one of (1) to (8) and (10) wherein the at least one compound selected from the group consisting of the EPA and the pharmaceutically acceptable salts and esters thereof is used in combination with the nicotinic acid or the pharmaceutically acceptable derivative thereof by administering the nicotinic acid or the pharmaceutically acceptable derivative thereof to a patient to which the at least one compound selected from the group consisting of the EPA and the pharmaceutically acceptable salts and esters thereof is administered.

(13) The ameliorating or therapeutic agent according to any one of (1) to (7) which is a kit comprising a preparation of the at least one compound selected from the group consisting of the EPA and the pharmaceutically acceptable salts and esters thereof and a preparation of the nicotinic acid or the pharmaceutically acceptable derivative thereof.

(14) The ameliorating or therapeutic agent according to any one of (1) to (13) wherein at least one compound selected from the group consisting of an antihyperlipidemic agent, an antioxidant, a blood flow improving agent, a bile acid derivative and components of food for specified health use and food with nutrient function claims is further used in combination as a third effective component.

(15) The ameliorating or therapeutic agent according to any one of (1) to (14) wherein at least one selected from the group consisting of polyenylphosphatidylcholine, unsaponifiable soybean oil (soysterol), y-orizanol, riboflavin butyrate, dextran sulfate sodium sulfur 18, pantethine, elastase, pravastatin, simvastatin, atorvastatin, fluvastatin, pitavastatin, rosuvastatin, cerivastatin, simfibrate, clofibrate, clinofibrate, bezafibrate, fenofibrate, orlistat, cetilistat, cholestyramine, colestimide, ezetimibe, vitamin C, vitamin E, N-acetylcysteine, probucol, cilostazol, ticlopidine hydrochloride, alprostadil, limaprost, isoxsuprine hydrochloride, batroxobin, dihydroergotoxine mesilate, traprost sodium hydrochloride, sarpogrelate hydrochloride, argatroban, naftidrofuryl lazoline, hepronicate, Shimotsu-to extract, ursodeoxycholic acid, chenodeoxycholic acid, powdered bile, deoxycholic acid, cholic acid, bile extract, bear bile, ox bile, dehydrocholic acid, biotin, cyanocobalamine, pantothenic acid, folic acid, thiamine, vitamin K, tyrosine, pyrodoxine, leucine, isoleucine and valine is further used in combination as the third effective component.

(16) The ameliorating or therapeutic agent according to (15) wherein the effective component is at least one selected from the group consisting of polyenylphosphatidylcholine, unsaponifiable soybean oil (soysterol), γ-orizanol, riboflavin butyrate, dextran sulfate sodium sulfur 18, pantethine, elastase, ursodeoxycholic acid, chenodeoxycholic acid, powdered bile, deoxycholic acid, biotin, cyanocobalamine, pantothenic acid, folic acid, thiamine, vitamin K, and tyrosine.

(17) The ameliorating or therapeutic agent according to any one of (1) to (16) which is in the form of an emulsion. In cases where the active ingredients are provided as separate preparations in this embodiment, at least one of them or only one of them may be in the form of an emulsion. Alternatively, both the components may be in the form of an emulsion.

(18) The ameliorating or therapeutic agent according to (17) which contains at least one emulsifier selected from the group consisting of:

(a) lecithin and optionally polyoxyethylene polyoxypropylene glycol;

(b) polyoxyethylene hydrogenated castor oil; and

(c) sucrose fatty acid ester.

(19) The ameliorating or therapeutic agent according to (18) wherein the lecithin in (a) is at least one selected from the group consisting of soybean lecithin, enzyme-degraded soybean lecithin, hydrogenated soybean lecithin and egg-yolk lecithin.

(20) The ameliorating or therapeutic agent according to (18) or (19) wherein the polyoxyethylene polyoxypropylene glycol in (a) is at least one selected from the group consisting of polyoxyethylene (3) polyoxypropylene (17) glycol, polyoxyethylene (20) polyoxypropylene (20) glycol, polyoxyethylene (42) polyoxypropylene (67) glycol, polyoxyethylene (54) polyoxypropylene (39) glycol, polyoxyethylene (105) polyoxypropylene (5) glycol, polyoxyethylene (120) polyoxypropylene (40) glycol, polyoxyethylene (160) polyoxypropylene (30) glycol, polyoxyethylene (196) polyoxypropylene (67) glycol, and polyoxyethylene (200) polyoxypropylene (70) glycol.

(21) An emulsified composition according to any one of (18) to (20) wherein the lecithin in (a) is soybean lecithin or enzyme-degraded soybean lecithin and the polyoxyethylene polyoxypropylene glycol is polyoxyethylene (105) polyoxypropylene (5) glycol.

(22) The ameliorating or therapeutic agent according to (18) or (22) wherein the polyoxyethylene hydrogenated castor oil in (b) is at least one selected from the group consisting of polyoxyethylene (20) hydrogenated castor oil, Polyoxyethylene (40) hydrogenated castor oil, polyoxyethylene (50) hydrogenated castor oil, polyoxyethylene (60) hydrogenated castor oil, and polyoxyethylene (100) hydrogenated castor oil.

(23) The ameliorating or therapeutic agent according to (18) or (22) wherein the polyoxyethylene hydrogenated castor oil in (b) is polyoxyethylene (60) hydrogenated castor oil.

(24) The ameliorating or therapeutic agent according to (18) wherein the sucrose fatty acid ester in (c) is at least one selected from the group consisting of sucrose laurate, sucrose myristate, sucrose palmitate, sucrose oleate, and sucrose stearate.

(25) The ameliorating or therapeutic agent according to any one of (17) to (24) wherein the emulsifier has a hydrophilic-lipophilic balance (hereinafter abbreviated as "HLB") of 10 to 18.

(26) The ameliorating or therapeutic agent according to any one of (17) to (25) wherein the emulsifier has an HLB of 12 to 16.

(27) The ameliorating or therapeutic agent according to any one of (17) to (26) wherein the EPA and the pharmaceutically acceptable salts and esters thereof are incorporated in amounts of 0.01 wt% to 50 wt%.

(28) The ameliorating or therapeutic agent according to any one of (17) to (27) wherein the EPA and the pharmaceutically acceptable salts and esters thereof are incorporated in amounts of 0.1 wt% to 30 wt%.

(29) The ameliorating or therapeutic agent according to any one of (17) to (28) wherein the EPA and the pharmaceutically acceptable salts and esters thereof are incorporated in amounts of up to 9 wt%.

(30) The ameliorating or therapeutic agent according to any one of (17) to (29) wherein the emulsifier is incorporated in an amount of 5 to 100 wt% with respect to 100 wt% of the EPA and the pharmaceutically acceptable salts and esters thereof.

(31) The ameliorating or therapeutic agent according to any one of (17) to (30) wherein the emulsifier is incorporated in an amount of 10 to 50 wt% with respect to 100 wt% of the EPA and the pharmaceutically acceptable salts and esters thereof.

(32) An emulsified composition according to (17) to (31) wherein the emulsifier is incorporated in an amount of up to 2 wt%.

(33) The ameliorating or therapeutic agent according to any one of (17) to (32) wherein the average emulsified droplet size is up to 2 μm.

(34) The ameliorating or therapeutic agent according to any one of (17) to (33) wherein the average emulsified droplet size in the ameliorating or therapeutic agent is up to 0.3 μm.

(35) A method for ameliorating or treating dyslipidemia which comprises a step of administering at least one compound selected from the group consisting of EPA and pharmaceutically acceptable salts and esters thereof; and a step of administering nicotinic acid or a pharmaceutically acceptable derivative thereof.

(36) The method according to (35) wherein the two administration steps are performed simultaneously.

(37) The method according to (35) wherein the two administration steps are performed at different periods.

(38) The method according to (35) wherein at least one value selected from the group consisting of serum total Cho, TG, LDLCho, HDLCho, VLDLCho, non-HDLCho, IDLCho, VHDLCho, free fatty acid, phospholipid, chylomicron, ApoB, Lp(a), RLPCho and sdLDLCho is measured or calculated, and administration is continued until the measured or calculated value falls within a normal range.

(39) A method for reducing side effects caused by nicotinic acid or a pharmaceutically acceptable derivative thereof, comprising a step of administering at least one compound selected from the group consisting of EPA and pharmaceutically acceptable salts and esters thereof; and a step of administering nicotinic acid or a pharmaceutically acceptable derivative thereof.

(40) The method according to (39) wherein the two administration steps are performed simultaneously.

(41) The method according to (39) wherein the two administration steps are performed at different periods.

(42) The method according to (39) wherein, when hot flash is observed or serum creatine phosphokinase (hereinafter abbreviated as "CPK") and the insulin resistance are measured and the side effects are seen, at least one selected from the group consisting of reducing the dose of the nicotinic acid or the pharmaceutically acceptable derivative thereof, interrupting administration of the nicotinic acid or the pharmaceutically acceptable derivative thereof, and increasing the dose of the EPA is performed until the side effects disappear or measured values fall within normal ranges.

(43) A food with health claims for humans having high blood lipid levels or for humans having poor peripheral blood circulation wherein at least one compound selected from the group consisting of EPA and pharmaceutically acceptable salts and esters thereof, and nicotinic acid or a pharmaceutically acceptable derivative thereof are used in combination as active ingredients .

EFFECTS OF INVENTION

By using at least one selected from the group consisting of EPA and pharmaceutically acceptable salts and esters thereof in combination with nicotinic acid or a pharmaceutically acceptable salt thereof, the present invention can provide an ameliorating or therapeutic agent for dyslipidemia which is safe and has high effective, and a method of using such an ameliorating or therapeutic agent.

More specifically, the ameliorating or therapeutic agent is expected to exhibit a synergistic ameliorating or therapeutic effect compared to the case where these components are used singly. The ameliorating or therapeutic agent is particularly expected to exhibit a synergistic ameliorating or therapeutic effect against dyslipidemia in improving plasma lipid markers such as blood total Cho, TG, LDLCho, non-HDLCho and HDLCho, increasing the peripheral skin temperature of the extremities decreased by peripheral arterial disease, prolonging the walking distance, or improving various symptoms such as joint pain, muscle fatigue, eye strain, numbness, coldness, pain and itching. The ameliorating or therapeutic agent is also expected to exhibit an ameliorating or therapeutic effect including reduction of Lp(a) in patients with dyslipidemia having a high Lp(a) level.

Nicotinic acid or its pharmaceutically acceptable derivative causes main side effects such as itching, hot flash and feeling of warmth at high frequencies, which are factors of reducing the drug compliance. Worsening of insulin resistance, worsening of liver damage, worsening of peptic ulcer and an elevated intraocular pressure have been reported, and nicotinic acid and its pharmaceutically acceptable derivative are carefully administered to patients with impaired liver function, peptic ulcer, glaucoma and diabetes. There is also a report that an increase in the serum CPK, muscle pain and rhabdomyolysis are easily caused by the combined use with statin. This invention can reduce the doses of the respective drugs, particularly nicotinic acid and its pharmaceutically acceptable derivative to reduce side effects such as itching, hot flash and feeling of warmth, thus leading to enhanced drug compliance. In particular, side effects such as an increased serum CPK, muscle pain and rhabdomyolysis can be reduced in patients having a history of any of the above symptoms or patients to which statin is being administered. Treatment can be continued in patients who could not be administered with nicotinic acid or its pharmaceutically acceptable derivative, or had no other choice than to interrupt the treatment because of the side effects.

The burden of the medication on patients can be reduced by providing a composite formulation or a kit. The ameliorating or therapeutic effect can be further increased by enhancing the drug compliance.

The emulsion improves the absorbability and therefore the effect of the present invention can also be exerted even when the drug is administered at a period other than during meals, after meals and just after meals, more specifically before meals, just before meals or before going to bed, when the drug is administered to patients with reduced intestinal absorptive capacity (elderly people, patients with an intestinal disorder, after intestinal surgery, terminal cancer patients, at the time of taking a lipase inhibitor), or when the dose is reduced.

Complementary and alternative medicine is increasing in developed countries such as Japan, US and European countries, and food for special dietary uses, food with health claims (food for specified health uses and food with nutrient function claims) and health food products (supplements) are used. Use of at least one selected from the group consisting of EPA and pharmaceutically acceptable salts and esters thereof in combination with nicotinic acid or a pharmaceutically acceptable derivative thereof enables provision of food with health claims which is safe and highly effective, is used for humans with high blood lipid levels (total Cho, TG, LDLCho, VLDLCho, non-HDLCho, IDLCho, free fatty acid, phospholipid, chylomicron, ApoB, Lp(a), RLPCho and sdLDLCho), for humans with low HDLCho and VHDLCho levels, or for humans with poor peripheral blood circulation. This invention can prevent humans with metabolic syndrome or would-be patients from further developing cardio-cerebrovascular events, peripheral ulcer of the extremities and gangrene, whereby the quality of life can be maintained.

DESCRIPTION OF EMBODIMENTS

The present invention is described below in detail.

The present invention provides an ameliorating or therapeutic agent for dyslipidemia which comprises at least one selected from the group consisting of EPA and pharmaceutically acceptable salts and esters thereof, and nicotinic acid or a pharmaceutically acceptable derivative thereof in combination as active ingredients . The present invention also provides a method of using such an ameliorating or therapeutic agent. The present invention provides an ameliorating or therapeutic agent which is a combined drug obtained by using at least one selected from the group consisting of EPA and pharmaceutically acceptable salts and esters thereof in combination with nicotinic acid or a pharmaceutically acceptable derivative thereof, a combined composition and a method of using the same.

In the present invention, the term "dyslipidemia" is a pathology in which at least one of blood lipid components does not fall within the normal range. Exemplary blood lipid components include total Cho, TG, LDLCho, HDLCho, VLDLCho, non-HDLCho, IDLCho, VHDLCho, free fatty acid, phospholipid, chylomicron, ApoB, Lp(a), RLPCho and sdLDLCho. In particular, total Cho, TG, LDLCho, non-HDLCho and HDLCho are clinically important lipid components and TG is preferred. Peripheral arterial disease associated with dyslipidemia is also included in the dyslipidemia as used in the present invention. Unless otherwise specified, the term "dyslipidemia" as used herein encompasses all of anomalies of the blood lipid components and peripheral arterial disease associated with dyslipidemia.

In the present invention, "peripheral arterial disease" is a pathology in which peripheral blood circulation is disrupted by any cause. Exemplary causes include ASO, Buerger's disease, Raynaud's disease, Raynaud's disease syndrome, intermittent claudication, artery embolism of the extremities, venous thrombosis, coldness of the extremities, thrombophlebitis, vibration disease, Takayasu's disease, diabetic retinopathy, frozen shoulder, air conditioning disease (deconditioning due to cooling such as feeling of cold and hot flash), alopecia, chilblain and frostbite. The peripheral arterial disease accompanies symptoms such as intermittent claudication, pain during walking, joint pain, muscle fatigue, eye strain, numbness, coldness, pain, pain at rest, cyanosis, flare, chilblain, shoulder stiffness, anemia, sallowness, pruritus and formication, and may lead to peripheral muscle atrophy of the extremities, ischemic ulcer and necrosis. Unless otherwise specified, the term "peripheral arterial disease" as used herein encompasses all of the above diseases, their symptoms and symptoms occurring when the diseases progressed.

EPA refers to all-cis-5,8,11,14,17-icosapentaenoic acid which is an ω-3 polyunsaturated fatty acid containing 20 carbon atoms, having 5 carbon-carbon double bonds in the molecule and having the first double bond at the third position from the methyl group side. Unless otherwise specified, the term "EPA" as used herein encompasses not only EPA but also pharmaceutically acceptable salts and esters thereof, amides, phospholipids, glycerides and other EPA derivatives.

EPA used in the present invention may be a synthetic, a semisynthetic or a natural product, or be in the form of natural oil containing such fatty acids. The natural product may mean one extracted from a natural oil containing EPA by a known method, one which has been further processed to produce a crude product or a further purified product. The semisynthetic product contains a polyunsaturated fatty acid produced by microorganisms and also includes ones obtained by subjecting such a polyunsaturated fatty acid or a natural polyunsaturated fatty acid to chemical treatments such as esterification and ester exchange. In the present invention, these may be used for EPA alone or in combination of two or more.

In the present invention, use may be made of EPA, more specifically EPA and pharmaceutically acceptable salts thereof including inorganic bases such as sodium salt and potassium salt; organic bases such as benzylamine salt and diethylamine salt; basic amino acid salts such as arginine salt and lysine salt; esters including alkyl esters such as ethyl ester and mono-, di- and triglycerides. Ethyl ester, that is, EPA-E is preferred.

The purity of EPA is not particularly limited but is preferably at least 25 wt%, more preferably at least 45 wt%, even more preferably at least 70 wt%, still more preferably at least 85 wt% and most preferably at least 96.5 wt% usually in terms of the EPA content in the total fatty acids of the composition of this agent.

The present composition may contain an $\omega$-3 polyunsaturated fatty acid other than EPA such as docosahexaenoic acid, docosapentaenoic acid, and $\alpha$-linolenic acid, or a pharmaceutically acceptable salt or ester thereof. A preferred example is EPA-E combined with DHA-E. In the case of using EPA-E and DHA-E, for example, the compositional ratio of EPA-E/DHA-E and the content ratio of EPA-E + DHA-E to the total fatty acids are not particularly limited as long as the purity of EPA in the composition is within the foregoing range. The compositional ratio of EPA-E/DHA-E is preferably at least 0.8, more preferably at least 1.0 and even more preferably at least 1.2. The purity of EPA-E + DHA-E is preferably high; for example, the content ratio of EPA-E + DHA-E to the total fatty acids and their derivatives is preferably at least 40 wt%, more preferably at least 55 wt%, even more preferably at least 84 wt% and most preferably at least 96.5 wt%. In other words, in this composition, the purity of the $\omega$-3 polyunsaturated fatty acids in the total fatty acids is preferably high; the purity of EPA + DHA which are $\omega$-3 polyunsaturated fatty acids is more preferably high; and the EPA purity is even more preferably high. The fatty acids other than the $\omega$-3 polyunsaturated fatty acids are preferably incorporated in small amounts; of long-chain unsaturated fatty acids, $\omega$-6 polyunsaturated fatty acids and particularly arachidonic acid are desirably incorporated in small amounts, preferably in amounts of less than 2 wt%, more preferably less than 1 wt%. An embodiment in which arachidonic acid is not substantially contained is most preferred.

Compared to fish oil or a fish oil concentrate, EPA-E that may be used for the ameliorating or therapeutic agent of the present invention has fewer impurities such as a saturated fatty acid and arachidonic acid which are not preferred to prevent cardiovascular events, and can exert an effect without problems of excessive nutritional intake and excessive intake of vitamin A. EPA-E is in the form of an ester and therefore has higher oxidation stability than fish oil which is mainly in a triglyceride form, and can forma sufficiently stable composition by the addition of a common antioxidant.

The soft capsule (Epadel from Mochida Pharmaceutical Co., Ltd.) containing high-purity EPA-E (at least 96.5 wt%) which is available as a drug for treating ASO and hyperlipidemia may be used in Japan for EPA-E. Lovaza (registered trademark): GlaxoSmithKline; soft capsule containing about 46.5 wt% of EPA-E and about 37.5 wt% of DHA-E) which is commercially available in the United States as a drug for treating hypertriglyceridemia may also be used as the mixture of EPA-E and DHA-E.

Purified fish oil may also be used for EPA. A monoglyceride, diglyceride or triglyceride derivative of EPA, or a combination thereof is also a preferred embodiment. Various products containing any of EPA, and salts and esters thereof are commercially available, as exemplified by Incromega F2250, F2628, E2251, F2573, TG2162, TG2779, TG2928, TG3525 and E5015 (Croda International PLC, Yorkshire, England)) and EPAX6000FA, EPAX5000TG, EPAX4510TG, EPAX2050TG, EPAX7010EE, K85TG, K85EE and K80EE (Pronova Biopharma, Lysaker, Norway)). These may also be purchased and used.

In the present invention, examples of the nicotinic acid and the pharmaceutically acceptable derivative thereof include nicotinic acid, niceritrol, nicomol, tocopherol nicotinate, nicotinic acid amide, and inositol hexanicotinate. Nicotinic acid and tocopherol nicotinate are preferred and tocopherol nicotinate is more preferred. Unless otherwise specified, the nicotinic acid derivative as used in the present invention encompasses all of nicotinic acid and its derivatives as described above.

In Japan, nicotinic acid is commercially available under the trade name of Nyclin (registered trademark) (Toa Eiyo Ltd.), niceritrol under the trade name of Perycit (registered trademark) (Sanwa Kagaku Kenkyusho Co., Ltd.), nicomol under the trade name of Cholexamin (registered trademark) (Kyorin Pharmaceutical Co., Ltd.), tocopherol nicotinate under the trade name of Juvela (registered trademark) N (Eisai Co., Ltd.), nicotinic acid amide under the trade name of nicotinamide Zonne (Torii Pharmaceutical Co., Ltd.), and inositol hexanicotinate under the trade name of Nicoxatin (registered trademark) (Fuso Pharmaceutical Industries, Ltd.). These may also be purchased and used. In terms of reducing side effects, it is also preferred to use a sustained-release agent of nicotinic acid (Niaspan, Kos Pharmaceuticals) commercially available in the United States. In addition, Advicor (registered trademark) (Kos Pharmaceuticals) and Simcor (registered trademark) (Abbott) are commercially available as composite formulation drugs combined with antihyperlipidemic agents, more specifically lovastatin and simvastatin, respectively. It is also preferred to use these drugs for patients with severe dyslipidemia.

In the foregoing drug of the present invention in which EPA and a nicotinic acid derivative are used in combination, a combination of EPA-E with nicotinic acid or tocopherol nicotinate is preferred and a combination of EPA-E with tocopherol nicotinate is particularly preferred.

In the present invention, the "combined use" of the active ingredients refers to using the active ingredients in combination, and includes administering a composite formulation containing both of EPA and a nicotinic acid derivative, and separately administering EPA and a nicotinic acid derivative as individual preparations at the same time or with a time lag. The embodiment of "separately administering EPA and a nicotinic acid derivative as individual preparations at the same time or with a time lag" includes Embodiment (1) in which a composition containing a nicotinic acid derivative as the effective component is administered to a patient to which EPA is administered, and Embodiment (2) in which a composition containing EPA as the effective component is administered to a patient to which a nicotinic acid derivative is administered. The "combined use" is not necessarily limited to the case where the two components simultaneously exist in the body of a patient, for example, in blood, but the "combined use" as used in the present invention refers to a use embodiment in which one drug is administered in a state in which the other drug works in the body of a patient. In this use embodiment, the effect of ameliorating or treating dyslipidemia is obtained by using the ameliorating or therapeutic agent of the present invention. A use embodiment in which the two components simultaneously exist in the body of a patient, for example, in blood is preferred. Another use embodiment in which one drug is administered to a patient and the other drug is administered within 24 hours from the administration of the first drug is also preferred.

The form of the combined use in the ameliorating or therapeutic agent of the present invention is not particularly limited as long as the active ingredients are combined, and includes a combined drug and a combined composition used in combination. The drug is provided in the following forms: (1) A single preparation obtained by simultaneously formulating the active ingredients is administered; (2) two preparations obtained by separately formulating the active ingredients are combined to form a kit or are separately provided without being combined and used for simultaneous administration by the same administration route; (3) two preparations obtained by separately formulating the active ingredients are combined to form a kit or are separately provided without being combined and administered with a time lag by the same administration route; (4) two preparations obtained by separately formulating the active ingredients are combined to form a kit or are separately provided without being combined and simultaneously administered by the different administration routes (administered to a single patient from different sites); and (5) two preparations obtained by separately formulating the active ingredients are combined to form a kit or are separately provided without being combined and administered with a time lag by the different administration routes (administered to a single patient from different sites).

Administration with a time lag includes, for example, a case in which EPA and a nicotinic acid derivative are administered in this order and administration in the reverse order. In the case of simultaneous administration, if the administration route is the same, both the drugs may be mixed just before the administration or separately administered. Alternatively, the drugs may be used for various purposes at different administration periods set in a planned manner. A specific example of the method includes one which involves administering one drug and administering the other drug at the period when the effect begins to be exerted or during the period when the effect is sufficiently exerted. Another method may be used in which one drug, particularly a nicotinic acid derivative is administered once a day while the other drug, particularly EPA is administered several times a day, for example, twice or three times a day, or likewise once a day. Administration of the two drugs once a day, and further simultaneous administration of the two drugs once a day or administration of the composite formulation once a day are preferred because the burden of the medication on patients is reduced to improve the drug compliance, whereby the ameliorating and therapeutic effect is expected to be increased while side effects are reduced. There is also a method in which both the drugs are administered and administration of one of them is stopped at the period when the effect begins to be exerted or during the period when the effect is sufficiently exerted. In cases where administration of the drug is stopped, the dose of the drug may be reduced in a stepwise fashion. For example, there is also a method in which one drug is administered during the drug holiday period when the other drug is not administered.

The use embodiment of the ameliorating or therapeutic agent for dyslipidemia according to the present invention is not limited as long as it is used in an embodiment in which the therapeutic effect is obtained by the combined use of at least EPA and a nicotinic acid derivative as the active ingredients . In addition to the ameliorating or therapeutic agent for dyslipidemia which is characterized by the use of only EPA and a nicotinic acid derivative, in other words, contains EPA and a nicotinic acid derivative in combination, this agent also includes one which is used in combination with other effective component.

An embodiment is preferred in which the therapeutic effect obtained by using EPA and a nicotinic acid derivative in combination is larger than the sum of the therapeutic effects obtained by separately using EPA and the nicotinic acid derivative at the same doses as used in the case of the combined use. The therapeutic effect as used herein is not particularly limited as long as it is an effect of improving biochemical markers associated with dyslipidemia or treating the pathology associated with dyslipidemia, or is an effect of controlling development of metabolic syndrome, cardio-cerebrovascular events, peripheral ulcer of the extremities and gangrene. For example, the therapeutic effect is to improve the concentrations of plasma lipid markers (total Cho, TG, LDLCho, HDLCho, VLDLCho, non-HDLCho, IDLCho, VHDLCho, free fatty acid, phospholipid, chylomicron, ApoB, Lp(a), RLPCho and sdLDLCho etc.), to increase the peripheral skin temperature of the extremities thatcan be measured by thermography, to prolong the walking distance, to improve inspection values such as elevated serum CPK level or to ameliorate various symptoms such as joint pain, muscle fatigue, eye strain, numbness, coldness, pain, pain at rest, itching, cyanosis, flare, chilblain, shoulder stiffness, anemia, sallowness, pruritus and formication. Biochemical or pathological parameters related to other diseases such as dyslipidemia and peripheral arterial disease may be used to monitor the ameliorating or therapeutic effect.

The dosage and administration period of EPA and the nicotinic acid derivative that may be used in the present invention are determined so as to be sufficient for the expression of the intended action and may be adequately increased or decreased depending on the dosage form, administration route, daily frequency, severity of the symptoms, body weight, age, and the like.

When orally administered, the ameliorating or therapeutic agent is administered at a dose in terms of EPA-E of 0.1 to 5 g/day, preferably 0.2 to 3 g/day, more preferably 0.4 to 1.8 g/day and even more preferably 0.6 to 0.9 g/day. The ameliorating or therapeutic agent may be administered if necessary in a single dose or in several divided doses although typically administered once to three times a day. The dose may be reduced depending on the dose of the nicotinic acid derivative. Absorption of EPA-E is affected by the meals and therefore EPA-E is preferably administered during or after meals and more preferably just after meals (within 30 minutes). In order to improve the absorbability, it is also preferred to emulsify EPA-E or to use it in combination with bile acid derivatives such as ursodeoxycholic acid, chenodeoxycholic acid, powdered bile, deoxycholic acid, cholic acid, bile extract, bear bile, ox bile and dehydrocholic acid. Emulsifying or combination use with such bile acid derivatives improves the absorbability and therefore the effect of the present invention can also be exerted even when the drug is administered at a period other than during meals, after meals and just after meals, more specifically before meals, just before meals or before going to bed, when the drug is administered to patients with reduced intestinal absorptive capacity (elderly people, patients with an intestinal disorder, after intestinal surgery, terminal cancer patients, at the time of taking a lipase inhibitor), or when the dose is reduced. When the drug is orally administered, the administration period is determined as appropriate for the severity of the symptoms and the degree of amelioration. Although not limited, the administration period is, for example, at least 1 year, preferably at least 2 years, more preferably at least 3.5 years, and even more preferably at least 5 years. Administration is desirably continued while the pathology associated with dyslipidemia or peripheral arterial disease, or abnormal biochemical index values continue, or while onset of dyslipidemia or peripheral arterial disease associated therewith, and/or a high risk of recurrence continues. For example, an embodiment in which the drug is administered every two days, or two or three times a week is also possible, and if necessary, drug holidays of about 1 day to about 3 months, and preferably about 1 week to about 1 month may be given.

The nicotinic acid derivative in the ameliorating or therapeutic agent of the present invention is preferably used according to the dosage regimen recommended for the drug used alone, and the dose may be adequately increased or decreased depending on the type, dosage form, administration route, daily frequency, severity of the symptoms, body weight, gender, age, and the like. When orally administered, the nicotinic acid derivative is administered once or twice a day at a dose in terms of nicotinic acid of 20 to 2000 mg/day, preferably 50 to 1000 mg/day and more preferably 100 to 500 mg/day, and at a dose in terms of tocopherol nicotinate of 6 to 600 mg/day, preferably 60 to 300 mg/day and more preferably 100 to 200 mg/day. However, the total dose may be divided into several smaller doses for administration. The nicotinic acid derivative may be administered once at bedtime to patients easily developing a side effect of hot flash.

Absorption of tocopherol nicotinate is affected by the meals and therefore tocopherol nicotinate is preferably administered during, after or just after meals. In order to improve the absorbability, it is also preferred to emulsify tocopherol nicotinate or to use it in combination with bile acid derivatives such as ursodeoxycholic acid, chenodeoxycholic acid, powdered bile, deoxycholic acid, cholic acid, bile extract, bear bile, ox bile and dehydrocholic acid. Emulsifying or combination use with such bile acid derivatives improves the absorbability and therefore the effect of the present invention can also be exerted even when the drug is administered at a period other than during meals, after meals and just after meals, more specifically before meals, just before meals or before going to bed, when the drug is administered to patients with reduced intestinal absorptive capacity (elderly people, patients with an intestinal disorder, after intestinal surgery, terminal cancer patients, at the time of taking a lipase inhibitor), or when the dose is reduced.

Depending on the physician's order, oral administration of the drug may be started at a lower dose than the recommended daily dose (for example, at a dose of 100 to 400 mg in the case of nicotinic acid) and continued at a dose gradually increased to the maximum daily dose (for example, 2000 mg in the case of nicotinic acid). The dose of the nicotinic acid derivative may be reduced depending on the dose of EPA. In terms of reducing side effects, it is more preferred to reduce the daily dose as much as possible and administer the drug once a day. It is even more preferred to use a sustained-release agent. It is also preferred to use in combination an agent for reducing hot flash symptoms caused by nicotinic acid (e.g., aspirin or laropiprant).

When the drug is orally administered, the administration period is determined as appropriate for the severity of the symptoms and the degree of amelioration. Although not limited, the administration period is, for example, at least 1 year, preferably at least 2 years, more preferably at least 3.5 years, and even more preferably at least 5 years. However, administration is desirably continued while the pathology associated with dyslipidemia or peripheral arterial disease, or abnormal biochemical index values continue, or while onset of dyslipidemia or peripheral arterial disease associated therewith, and/or a high risk of recurrence continues. For example, an embodiment in which the drug is administered every two days, or two or three times a week is also possible, and if necessary, drug holidays of about 1 day to about 3 months, and preferably about 1 week to about 1 month may be given.

In the present invention in which EPA is used in combination with a nicotinic acid derivative, it is also possible to set the dose of the EPA and/or that of the nicotinic acid derivative lower than typical doses commonly used. For example, it is also possible to use insufficient doses to obtain the therapeutic effect by using the individual drugs singly. This has an advantage that the side effects of the drugs and particularly the nicotinic acid derivative can be reduced.

An embodiment is also desirable in which the single dose of EPA and/or that of the nicotinic acid derivative are not sufficient to obtain the therapeutic effect, and the therapeutic effect obtained by using the EPA and the nicotinic acid derivative in combination is larger than the sum of the therapeutic effects obtained by separately using the EPA and the nicotinic acid derivative at the same doses as used in the case of the combined use.

An embodiment is also desirable in which the single dose of EPA and/or that of the nicotinic acid derivative are not sufficient to obtain the therapeutic effect, and the side effects caused by using the EPA and the nicotinic acid derivative in combination is smaller than the sum of the side effects caused by separately using the EPA and the nicotinic acid derivative at the same doses as used in the case of the combined use.

The single dose of EPA which is not sufficient to obtain the therapeutic effect varies with the individual condition, age, body weight and body type of a patient and is not limited. In the case of EPA-E and/or DHA-E, the daily dose is, for example, at least 0.1 g but less than 2 g, preferably at least 0.2 g but not more than 1.8 g, more preferably at least 0.3 g but not more than 0.9 g and even more preferably at least 0.3 g but not more than 0.6 g.

The single dose of the nicotinic acid derivative which is not sufficient to obtain the therapeutic effect varies with the individual condition, age, body weight and body type of a patient and is not limited. The daily dose of nicotinic acid is, for example, a recommended daily dose of less than 500 mg, preferably from 5 mg to 400 mg, more preferably from 10 mg to 200 mg, and even more preferably from 25 mg to 100 mg. The daily dose of tocopherol nicotinate is a recommended daily dose of less than 300 mg, preferably from 3 mg to 200 mg, more preferably from 6 mg to 100 mg and even more preferably from 15 mg to 50 mg.

The effect of the present invention is expected to be seen at a lower dose than that at which the effect of reducing the serum lipid level and the concomitant effect of ameliorating the peripheral arterial disease are seen by using the nicotinic acid derivative singly.

The dosage ratio of EPA to the nicotinic acid derivative is not particularly limited but the dosage ratio of EPA to nicotinic acid is 0.4 to 50:1, preferably 0.6 to 20:1, more preferably 0.8 to 10:1 and most preferably 1 to 3:1, and the dosage ratio of EPA to tocopherol nicotinate is 1 to 100:1, preferably 2 to 40:1, more preferably 2.5 to 20:1, and most preferably 3 to 6:1. In terms of reducing side effects of the nicotinic acid derivative, the dose of nicotinic acid or its pharmaceutically acceptable derivative may also be reduced to half to one-tenth. It is desirable to administer 500 to 2000 mg of nicotinic acid and 300 to 600 mg of tocopherol nicotinate to humans with respect to 1800 mg of EPA. EPA and the nicotinic acid derivative are desirably combined at such a ratio, even where a composite formulation is prepared.

The daily dose and administration frequency of and administration ratio between EPA and the nicotinic acid derivative may be increased or decreased by checking the concentrations of plasma lipid markers (total Cho, TG, LDLCho, HDLCho, VLDLCho, non-HDLCho, IDLCho, VHDLCho, phospholipid, chylomicron, ApoB, free fatty acid, Lp (a), RLPCho, sdLDLCho etc.), increased peripheral skin temperature of the extremities that can be measured by thermography, prolonged walking distance, inspection values such as elevated serum CPK or various symptoms such as joint pain, muscle fatigue, eye strain, numbness, coldness, pain, pain at rest, itching, cyanosis, flare, chilblain, shoulder stiffness, anemia, sallowness, pruritus and formication. For example, the therapeutic effect of the present invention can be obtained by the following process: the serum TG value is measured when the nicotinic acid derivative is administered alone; the dose of the nicotinic acid derivative is reduced based on the measured value and the administration of EPA is started. Occurrence of various side effects in the case of using the ameliorating or therapeutic agent of the present invention desirably does not exceed the frequency of occurrence of side effects such as hot flash as seen at a dose required in the single administration of the nicotinic acid derivative for obtaining the same therapeutic effect as in the present invention.

The ameliorating or therapeutic agent for dyslipidemia of the present invention may be administered either by administering the active ingredients (possibly with other inevitable components remaining after the purification) as it is, or by forming an adequate pharmaceutical preparation with a commonly used suitable carrier or medium, an excipient, a binder, a lubricant, a colorant, a flavor material, optionally sterilized water or a vegetable oil, or further a non-toxic organic solvent or a non-toxic solubilizing agent (for example, glycerin or propylene glycol), an emulsifier, a suspending agent (for example, Tween 80, gum arabic solution), an isotonic agent, a pH adjusting agent, a stabilizer, a soothing agent, a flavoring substance, a flavoring agent, a preservative, an antioxidant, a buffering agent, a colorant, an absorption promoter and other additives. Exemplary additives that may be incorporated include lactose, partly pregelatinized starch, hydroxypropylcellulose, macrogol, tocopherol, hydrogenated oil, sucrose fatty acid ester, hydroxypropyl methylcellulose, titanium oxide, talc, dimethylpolysiloxane, silicon dioxide, carnauba wax, and sodium desoxycholate.

In particular, EPA is highly unsaturated and therefore desirably contains an oil-soluble antioxidant, for example, at least one member selected from the group consisting of butylated hydroxytoluene, butylated hydroxyanisole, propyl gallate, propyl gallate, a pharmaceutically acceptable quinone, astaxanthin and $\alpha$-tocopherol in an effective amount. The emulsion containing moisture is easily oxidizable particularly by reducing the emulsion droplet size and therefore desirably contains a water-soluble antioxidant and/or an oil-soluble antioxidant in effective amounts, and preferably contains a water-soluble antioxidant and an oil-soluble antioxidant. Examples of the water-soluble antioxidant include ascorbic acid and derivatives thereof, erythorbic acid and derivatives thereof, nitrites and citric acid. Examples of the oil-soluble antioxidant include butylated hydroxytoluene, butylated hydroxyanisole, propyl gallate, propyl gallate, a pharmaceutically acceptable quinone, astaxanthin and $\alpha$-tocopherol. After the preparation, the emulsified composition is preferably nitrogen-purged, sealed and stored. The storage temperature is preferably room temperature and the composition is more preferably stored in a cool and dark space. Freezing may reduce the emulsion stability and cryopreservation is preferably avoided.

The dosage form of the preparation depends on the form of combination use of the active ingredients of the present invention and is not particularly limited. The preparation is administered to patients orally, intravenously, intra-arterially, intrarectally, intravaginally, externally or by inhalation. The preparation is orally administered in the dosage form of, for example, tablet, tablet film coating, capsule, microcapsule, granules, fine granules, powder, emulsion, self-emulsified preparation, oral liquid preparation, syrup, jelly or inhalant, and parenterally administered in the dosage form of, for example, external preparations such as ointment, suppository, (emulsifying, suspending or nonaqueous) injection, injectable solid emulsified or suspended just before use, infusion and transdermal preparation. A simple oral preparation is desirably administered to patients who can orally take the medicine. It is particularly preferred for the preparation to be orally administered in the dosage form of capsule such as soft capsule or microcapsule, tablet, or tablet film coating. An enteric-coated preparation or a sustained-release preparation may be orally administered, and it is also preferred to orally administer a jelly to dialysis patients and patients having difficulty swallowing.

When orally administered, the oral liquid preparations such as self-emulsified preparation and emulsion promote the absorption, thus enabling administration without limitation of the administration time while reducing the dose. In the case of using an emulsion, the emulsion preferably has a smaller droplet size. The average droplet size is up to 2 $\mu$m, preferably up to 1.5 $\mu$m, more preferably up to 0.3 $\mu$m, and even more preferably up to 0.2 $\mu$m.

Any emulsifier may be used if it is used for a pharmaceutical drug. Examples thereof include egg-yolk lecithin, soybean lecithin, egg-yolk phospholipid, soybean phospholipid, purified lanolin, glycerin, propylene glycol, polysorbate ester, monoglyceride, diglyceride and organic acid ester of the monoglyceride, polyoxyethylene polyoxypropylene glycol, polyoxyethylene hydrogenated castor oil, sucrose fatty acid ester, polyglycerin fatty acid ester, glycerin fatty acid ester, sorbitan fatty acid ester, propylene glycol fatty acid ester, sorbitan monostearate, sorbitan trioleate, sodium lauryl sulfate, and nonionic surfactants. Egg-yolk lecithin, soybean lecithin, polyoxyethylene polyoxypropylene glycol, sucrose fatty acid ester, sorbitan fatty acid ester, and propylene glycol fatty acid ester are preferred. Lecithin, and optionally polyoxyethylene polyoxypropylene glycol, polyoxyethylene hydrogenated castor oil, and sucrose fatty acid ester are more preferred. The emulsifier preferably has an HLB of 10 to 18 and more preferably 12 to 16. The emulsifiers may be incorporated alone or in combination of two or more.

The ameliorating or therapeutic agent may also contain an emulsifying aid, a stabilizer, a surfactant and an antioxidant. Examples of the emulsifying aid include fatty acids containing 12 to 22 carbon atoms such as stearic acid, oleic acid, linoleic acid, palmitic acid, linolenic acid, and myristic acid, or salts thereof. Examples of the stabilizer include phosphatidic acid, ascorbic acid, glycerin, cetanol, ethyl parahydroxybenzoate, propyl parahydroxybenzoate, trehalose, lactose, sucrose, glucose, and maltose. Examples of the surfactant include sucrose fatty acid ester, sorbitan fatty acid ester, glycerin fatty acid ester, polyglycerin fatty acid ester, polyoxyethylene sorbitan fatty acid ester, polyoxyethylene hydrogenated castor oil, polyoxyethylene alkyl ether, polyoxyethylene fatty acid ester, polyoxyethylene alkyl phenyl ether, polyoxyethylene polyoxypropylene glycol, and polyoxyethylene polyoxypropylene alkyl ether. Examples of the antioxidant include oil-soluble antioxidants such as butylated hydroxytoluene, butylated hydroxyanisole, propyl gallate, a pharmaceutically acceptable quinone, astaxanthin and $\alpha$-tocopherol and water-soluble antioxidants such as ascorbic acid and derivatives thereof, erythorbic acid and derivatives thereof, nitrites and citric acid.

EPA and/or the nicotinic acid derivative is incorporated in the emulsion in an amount of 0.1 to 60 wt%, preferably 1 to 40 wt%, and more preferably 3 to 30 wt%, and the emulsifier is incorporated in an amount of 0.1 to 10 wt%, preferably 0.2 to 5 wt% and more preferably 0.3 to 3 wt%.

The emulsion of the present invention may be manufactured by, for example, mixing and heating active ingredients , an emulsifier, glycerin, purified water and optionally other additives such as an antioxidant to prepare a solution, and by passing the solution through a common homogenizer, for example a pressurized jet homogenizer such as Manton Gaulin homogenizer about once to 50 times, preferably about twice to 20 times, for example, at a pressure of 50 to 700 kg/cm$^2$ to emulsify it, or by emulsifying the solution at 15000 rpm in a high-speed agitator (Bubbleless mixer available from Beryu Co., Ltd.), or by emulsifying the solution under high pressure at 20000 PSI in a high-pressure emulsification system (DeBee 2000 available from BEE International, Inc.), or by homogenizing the solution in a microfluidizer, high-speed thin-film spin mixer, high-pressure jet flow reverse emulsification system, or ultrasonic homogenizer. Such homogenization may be preceded by preliminary emulsion using a homomixer or other machine.

The food with health claims of the present invention may be manufactured by adding the active ingredients in the various dosage forms to food and mixing them, and then used. The food with health claims is preferably in the form of a syrup, jelly, emulsion, suspension, soft capsule, or microcapsule, and more preferably jelly, emulsion or microcapsule.

The ameliorating or therapeutic agent of the present invention may be formulated by any known method when two preparations obtained by separately formulating two drugs are used in combination. The ameliorating or therapeutic agent of the present invention may be a composite formulation containing EPA and a nicotinic acid derivative as its active ingredients .

A third drug may be incorporated as the effective component of the composite formulation. The third drug is not particularly limited but preferably does not diminish the effect of the present invention. Examples thereof include an antihyperlipidemic agent, an antioxidant, a blood flow improving agent, and a bile acid derivative.

Preferred examples of the third drug include antihyperlipidemic agents such as polyenylphosphatidylcholine, unsaponifiable soybean oil (soysterol), $\gamma$-orizanol, safflower oil (linoleic acid), riboflavin butyrate, dextran sulfate sodium sulfur 18, pantethine, and elastase. Other examples include statins such as pravastatin, simvastatin, atorvastatin, fluvastatin, pitavastatin, rosuvastatin, and cerivastatin; fibrate drugs such as simfibrate, clofibrate, clinofibrate, bezafibrate, and fenofibrate; lipolytic enzyme inhibitors such as orlistat and cetilistat; resins such as cholestyramine and colestimide; and ezetimibe.

Examples of the antioxidant include vitamins such as ascorbic acid (vitamin C) and tocopherol (vitamin E), N-acetylcysteine and probucol.

Examples of the blood flow improving agent include cilostazol, ticlopidine hydrochloride, alprostadil, limaprost, beraprost sodium, sarpogrelate hydrochloride, argatroban, naftidrofuryl, isoxsuprine hydrochloride, batroxobin, dihydroergotoxine mesilate, tolazoline hydrochloride, hepronicate and Shimotsu-to extract.

Examples of the bile acid derivative include ursodeoxycholic acid, chenodeoxycholic acid, powdered bile, deoxycholic acid, cholic acid, bile extract, bear bile, ox bile and dehydrocholic acid. Other preferred examples of the third drug include biotin (vitamin B7), cyanocobalamine (vitamin B12), pantothenic acid (vitamin B5), folic acid (vitamin B9), thiamine (vitamin B1), vitamin A, vitamin D, vitamin K, tyrosine, pyrodoxine (vitamin B6), branched-chain amino acids such as leucine, isoleucine and valine, and calcium, iron, zinc, copper and magnesium. Other examples include components of food for specified health use and food with nutrient function claims such as soybean protein, chitosan, low molecular weight sodium alginate, dietary fibers derived from psyllium seed coat, phospholipid-binding soybean peptide, plant sterol ester, plant stanol ester, diacylglycerol, globin obtained by proteolysis and green tea catechin.

The dosage form of the composite formulation is not particularly limited and the composite formulation is orally administered to patients in the dosage form of tablet, tablet film coating, capsule, microcapsule, granules, fine granules, powder, emulsion, self-emulsified preparation, oral liquid preparation, syrup or jelly, or parenterally administered in the dosage form of external preparations such as injection, infusion and transdermal preparation. For example, the composite formulation may be a sustained-release agent or a preparation of two drugs released with a time lag.

In addition to the active ingredients , the composite formulation of the present invention may also contain a pharmaceutically acceptable excipient. The composite formulation may appropriately contain known additives such as antioxidant, coating agent, gelling agent, flavoring substance, flavoring agent, preservative, emulsifier, pH adjusting agent, buffering agent and colorant. Preferred embodiments of the dosage form and excipient of the composite formulation are the same as those in the foregoing combined use.

The composite formulation of the present invention can be formulated according a common method. EPA powder is obtained by any known method such as a method described in JP H10-99046 which involves drying and pulverizing under high vacuum an oil-in-water emulsion containing (A) EPA-E, (B) dietary fibers, (C) a starch hydrolyzate and/or a poorly glycosylated reduced starch, and (D) a water-soluble antioxidant. Granules, fine granules, powder, a tablet, a tablet film coating, a chewable tablet, a sustained-release tablet, or an orally-disintegrating tablet (OD) may be obtained by a common method using EPA-E powder and nicotinic acid or its pharmaceutically acceptable derivative in powder form.

A chewable tablet may be obtained by any known method, for example, a method which involves emulsifying EPA-E in a water-soluble polymer solution such as hydroxypropyl methylcellulose to obtain an emulsified liquid, spraying the resulting emulsified liquid to an additive such as lactose to obtain a powder material (see JP H8-157362), mixing the powder material with a nicotinic acid derivative in power form and tableting them.

The sustained-release tablet is obtained, for example, by (1) forming one of EPA-E and a nicotinic acid derivative in the inner layer and the other in the outer layer, (2) forming disk-shaped matrices containing the respective components in two layers, (3) embedding a granular capsule containing one component in a matrix containing the other component, and (4) preliminarily mixing both the agents and then processing for sustained release. It is desirable to adjust the release rate of the respective active ingredients . Both the agents may be simultaneously released or individually released with a time lag.

The orally-disintegrating tablet and the film preparation for use in oral cavity may be manufactured by any known methods such as a technique described in JP H8-333243 and a technique described in JP 2005-21124 A, respectively. When a nicotinic acid derivative which does not simply dissolve in EPA is used to form, for example, a soft capsule or a liquid, is to be processed as described in Examples. The composite formulation of the present invention includes a preparation in which EPA and a nicotinic acid derivative are processed as described above to form a single formulation.

The composite formulation of the present invention is desirably released or absorbed so that the pharmacological effect of the active ingredients may be exhibited. The composite formulation of the present invention desirably has at least one of the following features: excellent releasing properties of the active ingredients , excellent absorbing properties of the active ingredients ; excellent dispersibility of the active ingredients ; excellent storage stability of the composite formulation; convenience of dosingand excellent compliance by a patient.

The ameliorating or therapeutic agent of the present invention is effective in ameliorating, treating or preventing reoccurrence of dyslipidemia in animals and particularly mammals, or in controlling the development of metabolic syndrome, cardio-cerebrovascular events, peripheral ulcer of the extremities and gangrene. Exemplary mammals include human, farm animals such as cow, horse and pig, and domestic animals such as dog, cat, rabbit, rat and mouse. Human is preferred. This drug is expected to exhibit a synergistic ameliorating or therapeutic effect against dyslipidemia in patients with dyslipidemia and particularly patients with metabolic syndrome who have an increased blood lipid level, show insulin resistance or have an increased blood pressure. Side effects can be reduced in patients who have concerns about side effects of the nicotinic acid derivative such as itching, hot flash and feeling of warmth, and other side effects such as reduced insulin resistance, worsened liver damage, worsened peptic ulcer, elevated intraocular pressure, elevated serum CPK, muscle pain and rhabdomyolysis, and have complications such as impaired liver function, peptic ulcer, glaucoma and diabetes, and patients taking statin in combination. Treatment can also be continued in patients who could not be administered with the nicotinic acid derivative or had no other choice than to interrupt the treatment with the nicotinic acid derivative because of such side effects.

The burden of the medication on patients can be reduced by providing a composite formulation or a kit. The ameliorating or therapeutic effect can be further increased

[EXAMPLES]

Next, the present invention is described in further detail, which by no means limit the scope of the present invention.

(Experimental Example 1)

Effectiveness in Cho diet rabbit

Pharmacological action of EPA-E and/or tocopherol nicotinate on plasma lipids, auricle skin temperature, and platelet aggregation is confirmed by using rabbits that have been fed with Cho diet (hereinafter referred to as "HCD diet") known to develop a dyslipidemia.

Japan white male rabbits (Funabashi Farm) having a body weight of 2.7 to 3.3 kg are allowed to freely take 100 g/day of normal diet (F-1, Funabashi Farm) or HCD diet (RC-4 supplemented with 1% Cho, Oriental Yeast Co.,Ltd.) for 12 weeks under 12 hour light-dark cycles at 23°C. 5 groups (each comprising 10 animals), namely, normal group (normal diet feeding); control group (HCD diet feeding); EPA-E group (HCD diet feeding + EPA-E administration); tocopherol nicotinate group (HCD diet feeding + tocopherol nicotinate administration); and combination group (HCD diet feeding + EPA-E administration + tocopherol nicotinate administration) are set. During the feeding, the EPA-E group is administered with 1000 mg/kg of EPA-E; the tocopherol nicotinate group is administered with 300 mg/kg of tocopherol nicotinate; and the combination group is administered with 1000 mg/kg of EPA-E and 30 mg/kg of tocopherol nicotinate after suspending in 5% aqueous solution of gum arabic. The administration is conducted orally once a day. The normal group and the control group are orally administered with 5% aqueous solution of gum arabic once a day. At the start of the administration and after 12 weeks of feeding, auricle skin temperature is measured by thermography and blood is collected for plasma biochemical tests. Platelet collagen aggregation is also checked after 12 weeks of feeding by measuring absorbance.

Compared to the normal group, the control group has significantly increased plasma total Cho and TG concentration, developing the dyslipidemia. The control group also exhibits decrease in the auricle skin temperature and enhances platelet collagen aggregation.

Compared to the control group, the EPA-E group and the tocopherol nicotinate group exhibits suppressed increase of plasma total Cho and TG concentration. Compared to the control group, the decrease of the auricle skin temperature and the enhancement of the platelet collagen aggregation are also suppressed.

The effect of suppressing the measurement values in the combination group is larger than the sum of the effects realized in each of the EPA-E group and tocopherol nicotinate group. Accordingly, the ameliorating or therapeutic agent of the present invention is useful for amelioration or treatment of the dyslipidemia and the associated disruption of peripheral blood circulation.

(Experimental Example 2)

Effectiveness in hypertriglyceridemia rat

6 week old male Zucker Diabetic Fatty rat (Charles River Japan) are allowed to freely take diabetes-inducing diet (Purina 5008 (crude protein content, 23.5%; crude lipid content, 6.5%)) under 12 hour light-dark cycles at 23°C. 4 groups (each comprising 10 animals), namely, control group; EPA-E group (EPA-E administration); tocopherol nicotinate group (tocopherol nicotinate administration); and combination group (EPA-E administration + tocopherol nicotinate administration) are set. During the feeding, the EPA-E group is administered with 300 mg/kg of EPA-E; the tocopherol nicotinate group is administered with 100 mg/kg of tocopherol nicotinate; and the combination group is administered with 300 mg/kg of EPA-E and 100 mg/kg of tocopherol nicotinate. The administration is conducted orally once a day. The drugs are administered by preparing an emulsion preparation according to Preparation Example 8. The control group is orally administered with base of the emulsion preparation of Preparation Example 8 once a day. At the start of the administration and after 4 weeks of feeding, auricle skin temperature is measured by thermography and blood is collected for plasma biochemical test.

After feeding 4 weeks, the control group exhibits significant increase in the plasma TG and free fatty acid concentration.

Compared to the control group, the EPA-E group and the tocopherol nicotinate group exhibits suppressed increase of plasma TG and free fatty acid concentration. The effect of suppressing the measurement values in the combination group is larger than the sum of the effects realized in each of the EPA-E group and the tocopherol nicotinate group. Accordingly, the ameliorating or therapeutic agent of the present invention is useful for amelioration or treatment of the dyslipidemia.

(Experimental Example 3)

Effctiveness in lauric acid-induced peripheral arterial occlusive hypertriglyceridemia rat

4 week old male Wister rats (Japan SLC, Inc.) are allowed to freely take high sucrose diet (AIN-76A, PURINA MILLS) for 4 weeks under 12 hour light-dark cycles at 23°C. 5 groups (each comprising 10 animals), namely, sham surgery group; control group, EPA-E group (EPA-E administration); tocopherol nicotinate group (tocopherol nicotinate administration); and combination group (EPA-E administration + tocopherol nicotinate administration) are set. During the feeding, the EPA-E group is administered with 300 mg/kg of EPA-E; the tocopherol nicotinate group is administered with 100 mg/kg of tocopherol nicotinate; and the combination group is administered with 300 mg/kg of EPA-E and 100 mg/kg of tocopherol nicotinate after suspending in 5% aqueous solution of gum arabic. The administration is conducted orally once a day. The sham surgery group and the control group are orally administered with 5% aqueous solution of gum arabic once a day. 2 weeks after the administration, the rat is anesthetized by intraperitoneally injecting the rat with 60 mg/kg of pentobarbital sodium. After the anesthetization, right femoral artery is exposed to inject 0.1 ml of aqueous solution of sodium laurate (10 mg/ml, pH 9.5). The site of the puncture is stanched with Aronalpha (Registered Trademark) (Toagosei Co.,Ltd.) and the incision is sutured. The sham surgery group is injected with 0.1 ml of physiological saline instead of the aqueous solution of sodium laurate. Progress of the lesion of the limbs on the peripheral side of the injection site is scored for wet gangrene and dry gangrene, and the lesion is visually inspected with naked eyes every day until 2 weeks after the surgery. Progress of the lesion is scored by the following criteria:

Score 0: no change, score 1: the lesion is limited to nail, score 2: the lesion is limited to finger, score 3: the lesion is expanded to footpad, and score 4: the lesion is expanded to lower limb.

2 weeks after the surgery, the score is "0" in the sham surgery group, while clear necrosis or deficiency lesion in the lower limb are found in the control group.

Compared to the control group, the EPA-E group and the tocopherol nicotinate group exhibits improved scores. The effect of suppressing the measurement values in the combination group is larger than the sum of the effects realized in each of the EPA-E group and tocopherol nicotinate group. Accordingly, the ameliorating or therapeutic agent of the present invention is useful for amelioration or treatment of the disruption of peripheral blood circulation associated with dyslipidemia.

(Experimental Example 4)

Patients with the diagnosis of dyslipidemia are divided into 3 groups (each group consisting of 15 patients). EPA-E group is administered with Epadel S (Registered Trademark) 900 (containing 900 mg of EPA-E) twice a day, namely, 1800 mg a day. Tocopherol nicotinate group is administered with Juvela N (Registered Trademark) soft capsule 200 mg (containing 200 mg of tocopherol nicotinate) one to three times a day; and combination group is administered with Epadel S (Registered Trademark) 900 twice a day, namely, 1800 mg a day and Juvela N (Registered Trademark) soft capsule 200 mg one to three times a day. Administration of the Juvela N (Registered Trademark) soft capsule 200 mg is started from one capsule a day, and after 2 weeks from the start of the administration, the dose is increased to twice a day and 2 capsules in total, and after 4 weeks from the start of the administration, the dose is increased to 3 times a day and 3 capsules in total. The dose is adequately adjusted depending on the conditions of the patient. At the start and 6 months after the administration, blood biochemistry tests such as plasma lipids, body fat ratio, and visceral fat level (body weight and body composition monitor HBF-361 manufactured by Omron), as well as abdominal circumference and lower limb skin temperature (thermography) are measured.

In all groups, the blood Cho and TG concentration decreases, and the plasma HDL Cho increases compared to the level before the surgery. The body fat ratio, the visceral fat level, and the abdominal circumference decreased despite increase in the lower limb skin temperature. Each index is synergistically improved in the combination group. In the combination group, increase in the dose of the Juvela N (Registered Trademark) soft capsule 200 mg is lower compared to the tocopherol nicotinate group, and the side effects such as hot flash are thereby suppressed. Accordingly, the ameliorating or therapeutic agent of the present invention is effective in ameliorating or treating dyslipidemia and the associated disruption of peripheral blood circulation, and also, for reducing the side effects such as development of the hot flash by tocopherol nicotinate.

(Experimental Example 5)

Effects of bile acid derivative on the absorption of EPA-E and tocopherol nicotinate

6 week old male Wister rats (Japan SLC, Inc.) are allowed to freely take normal diet (F-1, Funabashi Farm) under 12 hour light-dark cycles at 23°C. 3 groups (each comprising 3 animals), namely, control group; EPA-E + tocopherol nicotinate group (EPA-E administration + tocopherol nicotinate administration); and ursodeoxycholic acid combination group (EPA-E administration + tocopherol nicotinate administration + ursodeoxycholic acid administration) are set. After fasting one day, the EPA-E + tocopherol nicotinate group is administered with 300 mg/kg of EPA-E and 100 mg/kg of tocopherol nicotinate; the ursodeoxycholic acid combination group is administered with 300 mg/kg of EPA-E, 100 mg/kg of tocopherol nicotinate, and 10 mg/kg of ursodeoxycholic acid, after suspending in 5% aqueous solution of gum arabic. The control group is orally administered with 5% aqueous solution of gum arabic. Before the drug administration and 3 hours after the administration, blood is collected from neck vein and plasma EPA and tocopherol concentration are measured by gas chromatography.

In the control group, no change is observed in the plasma EPA and the tocopherol concentration before and after the administration. In the EPA-E + tocopherol nicotinate group, the plasma EPA and the tocopherol concentration increases. In the ursodeoxycholic acid combination group, increase in the plasma EPA and the tocopherol concentration is several times higher compared to the EPA-E + tocopherol nicotinate group. Accordingly, the combined use with the bile acid derivative is expected to promote the absorption of the ameliorating or therapeutic agent of the present invention and to improve the effects of the present invention.

(Experimental Example 6)

Effectiveness in high sucrose diet-fed hamster

Pharmacological action of EPA-E and/or tocopherol nicotinate on plasma lipids was confirmed by using hamsters that had been fed high sucrose diet.
6 week old male Syrian hamsters (Japan SLC, Inc.) were allowed to freely take fish meal-less F-1 diet (F-1, Funabashi Farm) under 12 hour light-dark cycles at 23°C for 2 weeks, and then, high sucrose diet (D11511, Research Diet Inc.) for 2 weeks. 6 groups (each comprising 6 animals), namely, control group, EPA-E 300 group, EPA-E 1000 group, tocopherol nicotinate 1000 group (hereinafter referred to as TN1000 group), low dose combination group, and high dose combination group were set.
During the feeding of the high sucrose diet, the EPA-E 300 group was administered with 300 mg/kg of EPA-E (Mochida Pharmaceutical Co.); the EPA-E 1000 group was administered with 1000 mg/kg of EPA-E; the TN1000 group was administered with 1000 mg/kg of tocopherol nicotinate (Wako Pure Chemical Industries, Ltd.); the low dose combination group was administered with 300 mg/kg of EPA-E and 1000 mg/kg of tocopherol nicotinate; and the high dose combination group was administered with 1000 mg/kg of EPA-E and 1000 mg/kg of tocopherol nicotinate after suspending in 5% aqueous solution of gum arabic. The control group was orally administered with 5% aqueous solution of gum arabic once a day.

Blood was collected one day before the start of the administration and on the 14th day of the administration, and plasma was separated by centrifugation. Plasma TG, total Cho, and HDL Cho concentration were respectively measured using a biochemical analyzer (A0400, Olympus Corporation) and commercially available assay reagent (Wako Pure Chemical Industries, Ltd.). The nan-HDL Cho concentration (total Cho-HDL Cho) was also calculated. No statistically significant difference was found in the plasma lipid concentrations of various groups on the day before the start of the administration.

The plasma TG, total Cho, and non-HDL Cho concentration of each group at 14th day of the drug administration was compared with the corresponding values of the control group, and change rate was calculated by the following equation:

$$\text{Change rate (\%)} = [\{(\text{lipid concentration of each group}) - (\text{lipid concentration of control group})\} \times 100] / (\text{lipid concentration of the control group})$$

The results are shown in Table 1. For the combination group, sum of the change rate of the EPA-E sole administration group and the change rate of the tocopherol nicotinate sole administration group is shown in Table 1 as the theoretical change rate of the combination group. When the change rate of the actual measurement was negative and the value was lower than the theoretical value, synergistic effect by the combined use in the lipid decrease was deemed to have been realized.

[Table 1]

| Group | | TG | Total Cho | Non-HDL Cho |
|---|---|---|---|---|
| EPA-E 300 | | 2.7 | 11.1 | 14.8 |
| EPA-E 1000 | | 19.6 | 2.7 | 11.1 |
| TN 1000 | | -23.3 | -3.7 | -11.6 |
| Low dose combination | Measured | -41.8 | -6.5 | -15.1 |
| | Theoretical | -20.6 | 7.4 | 3.2 |
| High dose combination | Measured | -46.8 | -16.2 | -18.9 |
| | Theoretical | -3.7 | -1.0 | -0.5 |

The lipid reducing action was not found for the EPA-E administration. In the TN1000 group, the action of reducing the TG and non-HDL Cho was found. In the combination group, all types of plasma lipids decreased, and also, the actually measured value was lower than the theoretical value of the combination.

In the hamsters fed on the high sucrose diet, the plasma lipid reducing action significantly larger than that realized in the TN1000 group was achieved in the combination group, and the synergistic effect of the combination was achieved by the administration of once a day while the dose used was the one insufficient for realizing the plasma lipid reducing effect in the case of the sole administration of the EPA-E. Accordingly, the ameliorating or therapeutic agent of the present invention has synergistic effects on the amelioration or treatment of the dyslipidemia and the associated disruption of peripheral blood circulation, and in particular, the combined use is useful since it enables reduction of the dose of the EPA-E and/or the tocopherol nicotinate as well as synergistic effect by the administration of once a day.

(Experimental Example 7)

Pharmacokinetics in beagle

EPA-E and tocopherol nicotinate were orally administered to male beagles (about 18 month old, Kitayama Labes Co.,Ltd.) (4 animals per group) after fasting, to monitor concentration of the EPA and the tocopherol nicotinate in blood. The test animal was fasted at least 18 hours before the drug administration, and each animal was administered with the composition of the amount corresponding to 600 mg of the EPA-E and 200 mg of the tocopherol nicotinate. The emulsion preparation administration group was administered with the emulsion preparation that had been prepared according to Preparation Example 8, and the control group was administered with composition comprising 1% of the EPA-E and 0.33% of the tocopherol nicotinate suspended in 5% aqueous solution of gum arabic. Blood was collected before the administration and 1, 2, 3, 4, 8, and 24 hours after the administration, and the plasma was separated and treated to measure plasma EPA concentration and tocopherol nicotinate concentration by LC/MS/MS and LC/MS, respectively.

From each blood concentration, maximum blood concentration ($C_{max}$), area under the curve of the blood concentration from 0 to 2 hours ($AUC_{0-2}$), and area under the curve of the blood concentration from 0 to 24 hours ($AUC_{0-24}$) were calculated. In the calculation of each parameter, the parameter was corrected by subtracting the blood concentration before the administration from the blood concentration.

In the emulsion preparation administration group, EPA and tocopherol nicotinate, and also $C_{max}$ and $AUC_{0-2}$ which are the parameter of absorption rate, and $AUC_{0-24}$ which is the parameter of the amount of absorption were all higher than those of the control group. For the EPA, $AUC_{0-2}$ which is the parameter for increase of the blood concentration immediately after the administration of the emulsion preparation was about 6 times higher than that of the control group, and similarly, $AUC_{0-24}$ was about twice higher than the control group. In the meanwhile, for the tocopherol nicotinate, $AUC_{0-2}$ of the emulsion preparation administration group was about 17 times higher than that of the control group, and similarly, $AUC_{0-24}$ was about 5 times higher than the control group. Under fasting conditions, absorption of the EPA and tocopherol nicotinate by the administration in the form of emulsion preparation was substantially equivalent with the case when EPA-E stock solution or Juvela N which is the tocopherol nicotinate preparation was administered under feeding conditions (see Japanese Pharmacology & Therapeutics, 1980, 2: 410-414). Furthermore, the time required to reach the maximum blood concentration ($T_{max}$) in the administration of tocopherol nicotinate by way of emulsion preparation under fating conditions was less than half the case when the Juvela N was administered under feeding conditions, and the absorption speed was also faster.

As demonstrated above, it was confirmed that the EPA-E and the tocopherol nicotinate are absorbed more rapidly and also at a higher amount when they are administered in the form of an emulsion preparation compared to the control group. Accordingly, the EPA-E and the tocopherol nicotinate emulsion preparation of the present invention is useful since the blood EPA concentration and the tocopherol nicotinate concentration increases at a higher rate and to a higher level to realize the pharmacological action more rapidly and more effectively, even if the drug is taken under fasting conditions, for example, before the meal or before going to the bed.

A composite formulation of the EPA-E and the nicotinic acid derivative is prepared by a method commonly used in the art.

(Preparation Example 1) Soft capsule preparation

[Table 2]

| <Preparation Example 1 (Soft capsule preparation)> | |
| --- | --- |
| Component | Composition |
| Ethyl icosapentaenoate | 300 mg |
| Nicotinic Acid | 100 mg |
| Gelatine | 170 mg |
| D-sorbitol | 25 mg |
| Conc. glycerine | 25 mg |
| Sodium hydroxide | Adequate amount |
| Purified water | Adequate amount |

Of the components of the composition of Table 2, above, the concentrated glycerin, the nicotinic acid, and the purified water are mixed, and after stirring the mixture, the sodium hydroxide is added for adjustment of the pH to approximately 7. The gelatin and the D-sorbitol are added to this solution, and the mixture is heated to 60°C and stirred for dissolution. This solution is defoamed under reduced pressure, and the purified water is added for viscosity adjustment to thereby obtain the solution for the capsule shell of the soft capsule preparation. This capsule shell solution for the soft capsule preparation and EPA-E are used to produce a soft capsule preparation containing 300 mg of EPA-E and 100 mg of nicotinic acid per capsule.

The procedure as described above is repeated to prepare the soft capsule preparations by replacing the nicotinic acid with 125 mg of niceritrol, 50 mg of nicomol, or 100 mg of inositol hexanicotinate.

(Preparation Example 2) Soft capsule preparation

[Table 3]

| <Preparation Example 2 (Soft capsule preparation)> | | |
|---|---|---|
| | Component | Composition |
| A | Ethyl icosapentaenoate | 300 mg |
| | Tocopherol nicotinate | 100 mg |
| B | Gelatine | 170 mg |
| | D-sorbitol | 25 mg |
| | Conc. glycerine | 25 mg |
| | Purified water | Adequate amount |

Water is added to the concentrated glycerin of the composition of Table 3, B, above, and then the gelatin and the D-sorbitol are added to this solution, and the mixture is heated to 60°C and stirred for dissolution. This solution is defoamed under reduced pressure, and the purified water is added for viscosity adjustment to thereby obtain the solution for the capsule shell of the soft capsule preparation. The EPA-E and the tocopherol nicotinate of the composition A, above, are heated and stirred at 40°C to obtain the solution for the content of the soft capsule preparation. This capsule shell solution for the soft capsule preparation and the soft capsule preparation content solution are used to produce a soft capsule preparation containing 300 mg of EPA-E and 100 mg of tocopherol nicotinate per capsule.

The procedure as described above is repeated to produce a soft capsule further containing 5 mg of ursodeoxycholic acid of 1 mg of $\alpha$-tocopherol in addition to the components of the composition A.

(Preparation Example 3) Liquid preparation

[Table 4]

| <Preparation Example 3 (Liquid preparation)> | | |
|---|---|---|
| | Component | Composition |
| A | Ethyl icosapentaenoate | 1800 mg |
| | Tocopherol nicotinate | 600 mg |
| | Orange oil | 81 mg |
| B | Polyoxyethylene (105) polyoxypropylene (5) glycol | 144 mg |
| | Trehalose | 1350 mg |
| | Ascorbic Stearate | 1.8 mg |
| | Sodium erysorbate | 117 mg |
| C | Sodium hydroxide | Adequate amount |
| | Purified water | Adequate amount |
| Total | | 9 g |

Purified water is added to the components of the composition of Table 4, B, above, for dissolution, and sodium hydroxide is added to adjust the pH to approximately 7. Components of A are added to this solution, and the mixture is stirred at high speed under reduced pressure. 1.5 g of the resulting emulsion is dispensed in an aluminum laminate film stick package, and after purging the interior of the package with nitrogen, the package is sealed to prepare a liquid preparation containing 300 mg of EPA-E and 100 mg tocopherol nicotinate per package.

The procedure as described above is repeated to produce a liquid preparation further containing 5 mg of ursodeoxycholic acid in addition to the components of the composition A.

(Preparation Example 4) Jelly preparation

[Table 5]

| <Preparation Example 4 (Jelly preparation)> | | |
|---|---|---|
| | Component | Composition |
| A | Ethyl icosapentaenoate | 1800 mg |
| | Orange oil | 81 mg |
| B | Nicotinic acid | 500 mg |
| | Polyoxyethylene (105) polyoxypropylene (5) glycol | 144 mg |
| | Trehalose | 1350 mg |
| | Ascorbic Stearate | 1.8 mg |
| | Sodium erysorbate | 117 mg |
| | pullulan | 270 mg |
| C | Carrageenan | 37.8 mg |
| | Carob bean gum | 22.5 mg |
| | Conc. Glycerine | 675 mg |
| D | Sodium hydroxide | Adequate amount |
| | Purified water | Adequate amount |
| Total | | 9 g |

The purified water is added to the components of the composition of Table 5, B, above, for dissolution, and the sodium hydroxide is added to adjust the pH to approximately 7. Components of the composition A are added to this solution, and the mixture is stirred at high speed under reduced pressure to form an emulsion. The resulting emulsion is heated to 85°C, and to this emulsion is added a dispersion of the components of the composition C which has been stirred for homogeneous dispersion. The mixture is homogeneously mixed, and 4.5 g of the resulting preparation is dispensed in an aluminum laminate film stick package, and after purging the interior of the package with nitrogen, the package is sealed and cooled for solidification to thereby prepare a jelly preparation containing 900 mg of EPA-E and 250 mg nicotinic acid per package.

The procedure as described above is repeated by replacing the nicotinic acid with 200 mg of tocopherol nicotinate, 400 mg of niceritrol, 200 mg of nicomol, or 400 mg inositol hexanicotinate to prepare the jelly preparation.

(Preparation Example 5) Composite formulation package

Seamless soft capsules having a gelatin film with the diameter of about 4 mm and containing 20 mg of EPA-E or 20 mg of tocopherol nicotinate are prepared. 45 capsules of the EPA-E-containing seamless soft capsule and 15 capsules of tocopherol nicotinate are placed in an aluminum laminate film stick package, and the interior is purged with nitrogen. The package is sealed to prepare a composite formulation package containing 900 mg of EPA-E and 300 mg of tocopherol nicotinate per package.

1 tablet of Niaspan (Registered Trademark) 500 mg tablet (containing 500 mg of nicotinic acid per tablet) and 90 capsules of the EPA-E-containing seamless soft capsule are placed in an aluminum laminate film stick package, and the interior is purged with nitrogen. The package is sealed to prepare a composite formulation package containing 1800 mg of EPA-E and 500 mg of nicotinic acid per package.

(Preparation Example 6) Emulsion preparation

3.6 g of phospholipid, 0.15 g of sodium oleate, and 0.15 g of phosphatidic acid are added to 9 g of EPA-E and 1 g of tocopherol nicotinate, and the mixture is dissolved by heating to 40 to 75°C. To this mixture, 200 ml of purified water, and then, 7.5 g of glycerin is added, and purified water at 20 to 40°C is added to a total volume of 300 ml. This mixture is preliminarily emulsified by using a homomixer, and then emulsified by passing ten times through Manton-Gaulin Homogenizer at first stage pressure of 120 kg/cm$^2$ and total pressure of 500 kg/cm$^2$ to thereby obtain an emulsion having an average particle diameter of up to 0.2 $\mu$m with improved storage stability. This emulsion is dispensed in ampules at 30 ml per ampule to thereby produce an emulsion preparation containing 900 mg of EPA-E and 100 mg of tocopherol nicotinate per ampule.

(Preparation Example 7) Emulsion preparation

100 g of $\omega$3 fatty acid (Lovaza (Registered Trademark) (K85EE) containing about 90% of $\omega$3 fatty acid and about 84% of EPA-E + DHA-E with the EPA-E : DHA-E of about 1.2 : 1), 5 g of tocopherol nicotinate, 25 g of concentrated glycerin, and 12 g of egg yolk phospholipid are weighed, and injection grade distilled water is added to 1 liter, and this mixture is dispersed by using a homomixer. This dispersion is emulsified by passing through Manton-Gaulin Homogenizer at the first stage pressure of 100 kg/cm$^2$ and second stage pressure of 50 kg/cm$^2$ to thereby obtain an emulsion having an average particle diameter of up to 1 $\mu$m having improved storage stability. This emulsion is dispensed in glass bottles at 40 ml/bottle to thereby produce an emulsion preparation containing about 3.36 g of EPA-E + DHA-E and 200 mg of tocopherol nicotinate per bottle.

(Preparation Example 8) Emulsion preparation

80.0 g of concentrated glycerin and 80.0 g of purified water were added to 2.4 g of soybean lecithin, and the mixture was stirred. To this mixture, a solution of 5.3 g of tocopherol nicotinate in 16.0 g of EPA-E was incrementally added with stirring (Preparation A). In the meanwhile, 20.8 g of sodium erysorbate, 160.0 g of trehalose, and 2.4 g of polyoxyethylene (105) polyoxypropylene (5) glycol were dissolved in 1233.1 g of purified water (Preparation B). Next, Preparation B was incrementally added to the Preparation A with stirring, and this mixture was emulsified by using a high speed homogenizer (Bubbleless Mixer manufactured by BeRyu Co.,Ltd.) at 15000 rpm to produce an emulsion. Next, this emulsion was emulsified at a high pressure of 20000 PSI by using a high-pressure emulsifier (DeBee 2000 manufactured by BEE International, Inc.) to prepare an emulsified composition. The resulting emulsified composition was stored at room temperature until the evaluation in a container which was purged with nitrogen. Formulation of the emulsified composition is shown in Table 6 (% by weight).

[Table 6]

| <Preparation Example 8 (Emulsion preparation)> | |
| --- | --- |
| Component | Formulation (% by weight) |
| EPA-E | 1.00 |
| Tocopherol nicotinate | 0.33 |
| Soybean lecithin | 0.15 |
| Polyoxyethylene (105) polyoxypropylene (5) glycol | 0.15 |
| Sodium erysorbate | 1.30 |
| Conc. Glycerine | 5.00 |
| Trehalose | 10.00 |
| Purified water | Adequate amount |
| Total | 100.00 |

The emulsified composition was measured for its average drop diameter (volume average diameter) by using a particle size analyzer (Nanotrac manufactured by Nikkiso Co., Ltd.) using water for the dispersion medium. The average drop diameter of the as-prepared emulsified composition was 0.20 μm. The average drop diameter of the emulsified composition with lapse of time is shown in Table 7. The emulsified composition had excellent storage stability with no change in its average drop diameter after storing in a refrigerator (at 2 to 10°C), at 25°C, and at 40°C for 4 weeks.

[Table 7]

| Storage conditions | Volume average diameter (μm) | | | |
|---|---|---|---|---|
| | Initial | 1 week | 2 weeks | 4 weeks |
| Refrigerator (at 2 to 10°C) | | 0.19 | 0.20 | 0.20 |
| 25°C | 0.20 | 0.19 | 0.19 | 0.19 |
| 40°C | | 0.19 | 0.19 | 0.20 |

This emulsified composition was dispensed in glass bottles at 100 ml per bottle to produce an emulsion preparation containing 1000 mg of EPA-E and 330 mg of tocopherol nicotinate per bottle.

(Preparation Example 9) Emulsion preparation

4.8 g of polyoxyethylene (60) hydrogenated castor oil, 20.8 g of sodium erythorbate, 80.0 g of concentrated glycerin, 160.0 g of trehalose, and 1313.1 g of purified water are weighed and dissolved. To this mixture is added 16.0 g of Lovaza (Registered Trademark) (K85EE) having 5.3 g of tocopherol nicotinate dissolved therein. This mixture is emulsified by the procedure described in Preparation Example 8 to produce an emulsion with an average particle diameter of up to 0.3 μm having excellent storage stability. This emulsion is dispensed in glass bottles at 50 ml per bottle to obtain emulsion preparation containing about 420 mg of EPA-E + DHA-E and 165 mg of tocopherol nicotinate per bottle. Formulation of the emulsified composition is shown in Table 8 (% by weight).

[Table 8]

| <Preparation Example 9 (Emulsion preparation)> | |
|---|---|
| Component | Formulation (% by weight) |
| Lovaza | 1.00 |
| (content of EPA-E + DHA-E) | (about 0.84) |
| Tocopherol nicotinate | 0.33 |
| polyoxyethylene (105) polyoxypropylene (5) glycol | 0.3 |
| Sodium erysorbate | 1.30 |
| Conc. Glycerine | 5.00 |
| Trehalose | 10.00 |
| Purified water | Adequate amount |
| Total | 100.00 |

(Preparation Example 10)

3.84 g of sucrose fatty acid ester (Surfhope SE PHARMA J-1816, Mitsubishi-Kagaku Foods Corporation), 0.96 g of sucrose fatty acid ester (Surfhope SE PHARMA J-1805, Mitsubishi-Kagaku Foods Corporation), 20.8 g of sodium erysorbate, 80.0 g of concentrated glycerin, 160.0 g of trehalose, and 1313.1 g of purified water are weighed and heated to 70°C for dissolution. To this mixture is incrementally added solution of 5.3 g of tocopherol nicotinate in 16.0 g of EPA-E with stirring. This mixture is emulsified by the procedure described in Preparation Example 8 to prepare an emulsified composition with an average particle diameter of up to 0.3 μm having excellent storage stability. The resulting composition is stored at room temperature until the evaluation in a container which is purged with nitrogen. Formulation of the emulsified composition is shown in Table 9 (% by weight). This emulsified composition is dispensed in ampules at 60 ml per ampule to produce an emulsion preparation containing 600 mg of EPA-E and about 200 mg of tocopherol nicotinate per ampule.

[Table 9]

| <Preparation Example 10 (Emulsion preparation)> | |
|---|---|
| Component | Formulation (% by weight) |
| EPA-E | 1.00 |
| Tocopherol nicotinate | 0.33 |
| Sucrose fatty acid ester (J-1816) | 0.24 |
| Sucrose fatty acid ester (J-1805) | 0.06 |
| Sodium erysorbate | 1.30 |
| Conc. Glycerine | 5.00 |
| Trehalose | 10.00 |
| Purified water | Adequate amount |
| Total | 100.00 |

Compared to the case where the respective components are used singly, the ameliorating or therapeutic agent for dyslipidemia in the present invention in which EPA and a nicotinic acid derivative are used in combination as active ingredients is expected to exhibit a synergistic ameliorating or therapeutic effect against dyslipidemia and peripheral arterial disease associated therewith. In particular, the ameliorating or therapeutic agent is expected to exhibit a synergistic ameliorating or therapeutic effect against dyslipidemia and peripheral arterial disease associated therewith in patients with hypercholesterolemia, hypertriglyceridemia, high LDL cholesterolemia and low HDL cholesterolemia, patients with advanced arteriosclerosis, and patients with ASO, Buerger's disease, Raynaud's disease, and Raynaud's disease syndrome. Even in patients with less advanced arteriosclerosis or having no complications such as ASO, Buerger's disease, Raynaud's disease and Raynaud's disease syndrome, the pathology is expected to be considerably ameliorated by the combination use with a small amount of the nicotinic acid derivative. The ameliorating or therapeutic agent is also expected to exhibit an ameliorating or therapeutic effect including reduction of Lp(a) in patients with dyslipidemia having a high Lp(a) level.

The present invention is capable of reducing the doses of the respective active ingredients used singly. In particular, sides effects can be reduced in patients administered with statin who have concerns about severe side effects such as hot flash and rhabdomyolysis which may be caused by the nicotinic acid derivative at high frequencies. In addition, treatment can be continues in patients who could not be administered with the nicotinic acid derivative or had no other choice than to interrupt the treatment because of such side effects.

The burden of the medication on patients can be reduced by providing a composite formulation or a kit. The ameliorating or therapeutic effect can be further increased by enhancing the drug compliance.

The emulsion improves the absorbability and therefore the effect of the present invention can also be exerted even when the drug is administered at a period other than during meals, after meals and just after meals, more specifically before meals, just before meals or before going to bed, when the drug is administered to patients with reduced intestinal absorptive capacity (elderly people, patients with an intestinal disorder, after intestinal surgery, terminal cancer patients, at the time of taking a lipase inhibitor), or when the dose is reduced.

## Claims

1. An ameliorating or therapeutic agent for dyslipidemia comprising, as active ingredients to be applied in combination, at least one compound selected from the group consisting of icosapentaenoic acid and pharmaceutically acceptable salts and esters thereof and tocopherol nicotinate.

2. An ameliorating or therapeutic agent according to claim 1 wherein the dyslipidemia is at least one member selected from hypercholesterolemia, hypertriglyceridemia, high low density lipoprotein cholesteremia, high non-high density lipoprotein cholesteremia, and low high density lipoprotein cholesteremia.

3. An ameliorating or therapeutic agent according to claim 1 or 2 wherein the at least one compound selected from the group consisting of icosapentaenoic acid and pharmaceutically acceptable salts and esters thereof is ethyl icosapentaenoate.

4. An ameliorating or therapeutic agent according to any one of claims 1 to 3 wherein the agent is a composite formulation of at least one compound selected from the group consisting of icosapentaenoic acid and pharmaceutically acceptable salts and esters thereof and tocopherol nicotinate.

5. An ameliorating or therapeutic agent according to any one of claims 1 to 4 wherein the agent is in the form of an emulsion preparation.

## INTERNATIONAL SEARCH REPORT

International application No.

PCT/JP2009/062352

| A. CLASSIFICATION OF SUBJECT MATTER |
| --- |
| See extra sheet. |

According to International Patent Classification (IPC) or to both national classification and IPC

| B. FIELDS SEARCHED |
| --- |
| Minimum documentation searched (classification system followed by classification symbols)<br>A61K31/455, A61K9/10, A61K31/202, A61K31/232, A61P3/06,<br>A61P7/06, A61P9/00, A61P9/08, A61P9/10, A61P9/14, A61P17/02, A61P17/04,<br>A61P17/14, A61P21/02, A61P25/04, A61P27/02, A61P43/00 |

| Documentation searched other than minimum documentation to the extent that such documents are included in the fields searched |
| --- |
| Jitsuyo Shinan Koho        1922–1996   Jitsuyo Shinan Toroku Koho   1996–2009<br>Kokai Jitsuyo Shinan Koho    1971–2009   Toroku Jitsuyo Shinan Koho   1994–2009 |

| Electronic data base consulted during the international search (name of data base and, where practicable, search terms used) |
| --- |
| CA/MEDLINE/EMBASE/BIOSIS(STN), JSTPlus/JMEDPlus/JST7580(JDreamII) |

| C. DOCUMENTS CONSIDERED TO BE RELEVANT |
| --- |

| Category* | Citation of document, with indication, where appropriate, of the relevant passages | Relevant to claim No. |
| --- | --- | --- |
| X<br>Y | Yutaka OSAWA, "Ensan Sarpogrelate Fukuyo de ADL ga Kaizen shita Tonyobyo o Genshikkan to suru Toseki Kanja no 1-rei", Angiology Frontier, 2007, Vol.6, pages 171 to 175 | 1-3<br>4,5 |
| X<br>Y | Satoshi TAKEDA et al., "Simvastatin, ethyl icosapentate, tocopherol nicotinate Sanzai Heiyo Ryoho ni yoru Kyusei Shinkin Kosoku Go Saikyosaku Yokusei Koka no Kento", Yamanashi Igaku, 2000, Vol.28, page 272 | 1-3<br>4,5 |
| X<br>Y | Masami HOSHINO et al., "5-gata Ko-lipo Tanpaku Kessho ni yoru Kyusei Suien o Kurikaeshita 1-rei", Dai 40 Kai Nippon Fukubu Kyukyu Igakukai Sokai Shoroku, 2004, page 532, Shi-233 | 1-3<br>4,5 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>21 August, 2009 (21.08.09) | Date of mailing of the international search report<br>01 September, 2009 (01.09.09) |
| --- | --- |
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

| | International application No. |
|---|---|
| | PCT/JP2009/062352 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 59-172416 A  (Terumo Corp.),<br>29 September, 1984 (29.09.84),<br>Claims 1, 2; examples 1, 2<br>& US 5034414 A          & GB 2139889 A<br>& DE 3409793 A          & FR 2542613 A<br>& BE 899184 A          & SE 8401393 A | 4,5 |
| Y | JP 2007-197328 A  (Nippon Fine Chemical Co., Ltd.),<br>09 August, 2007 (09.08.07),<br>Claims 1, 3, 5<br>(Family: none) | 4,5 |

Form PCT/ISA/210 (continuation of second sheet) (April 2007)

**INTERNATIONAL SEARCH REPORT**

International application No.

PCT/JP2009/062352

Continuation of A. CLASSIFICATION OF SUBJECT MATTER
  (International Patent Classification (IPC))

A61K31/455(2006.01)i, A61K9/10(2006.01)i, A61K31/202(2006.01)i,
A61K31/232(2006.01)i, A61P3/06(2006.01)i, A61P7/02(2006.01)i,
A61P7/06(2006.01)i, A61P9/00(2006.01)i, A61P9/08(2006.01)i,
A61P9/10(2006.01)i, A61P9/14(2006.01)i, A61P17/02(2006.01)i,
A61P17/04(2006.01)i, A61P17/14(2006.01)i, A61P21/02(2006.01)i,
A61P25/04(2006.01)i, A61P27/02(2006.01)i, A61P43/00(2006.01)i

        (According to International Patent Classification (IPC) or to both national
        classification and IPC)

Form PCT/ISA/210 (extra sheet) (April 2007)

**INTERNATIONAL SEARCH REPORT**

| International application No. |
| --- |
| PCT/JP2009/062352 |

---

**Box No. II       Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

**Box No. III       Observations where unity of invention is lacking (Continuation of item 3 of first sheet)**

This International Searching Authority found multiple inventions in this international application, as follows:
    The inventions of claims 1-3 are not novel, as disclosed in Documents 1-3.  Therefore, these inventions have no special technical feature.

    Document 1: Yutaka OSAWA, Angiology Frontier,2007, Vol.6, pages 171 to 175
    Document 2: Satoshi TAKEDA et al., Yamanashi Igaku, 2000, Vol.28,page 272
    Document 3: Masami HOSHINO et al., Dai 40 Kai Nippon Fukubu Kyukyu Igakukai Sokai Shoroku, 2004, page 532, Shi-233

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H1099046 B
- JP H8157362 B
- JP 2005021124 A

**Non-patent literature cited in the description**

- Nanzando's Medical Dictionary. Nanzando Co., Ltd., March, 2006, 2265
- Guideline for Prevention of Atherosclerotic Cardiovascular Diseases. Kyowa Kikaku Ltd, 25 April 2007
- Juvela N, Drug interview form. Eisai Co., Ltd, February 2008
- **Epadel S.** Drug interview form. Mochida Pharmaceutical Co., Ltd, March 2007
- *Angiology Frontier,* 2007, vol. 6, 171-175
- Japanese Circulation Journal. 2000, vol. 64, 309
- *Abstract of the 40th Annual Congress of Japanese Society for Abdominal Emergency Medicine,* 2004, 532
- Japanese Pharmacology & Therapeutics. 1980, vol. 2, 410-414